# EUROPÄISCHE PATENTSCHRIFT

(11) **EP 1 648 950 B1**
(45) Veröffentlichungstag und Bekanntmachung des Hinweises auf die Patenterteilung: **13.06.2007**
(21) Anmeldenummer: 04766243.2
(22) Anmeldetag: 16.07.2004
(51) Int. Cl.: C08G 18/10, C08G 18/28, C08G 59/28

(54) **HITZEHÄRTENDE ZUSAMMENSETZUNGEN MIT TIEFTEMPERATUR-SCHLAGZÄHIGKEITSMODIFIKATOREN**
THERMOHARDENING COMPOSITIONS COMPRISING LOW-TEMPERATURE IMPACT STRENGTH MODIFIERS
COMPOSITIONS THERMODURCISSABLES COMPRENANT DES MODIFICATEURS DE RESISTANCE AUX CHOCS AGISSANT A BASSES TEMPERATURES

(30) Priorität: 16.07.2003 EP 03016146
(43) Veröffentlichungstag der Anmeldung: 26.04.2006
(73) Patentinhaber: Sika Technology AG, 6340 Baar (CH)
(72) Erfinder: KRAMER, Andreas, CH-8006 Zürich (CH); FINTER, Jürgen, CH-8037 Zürich (CH); GERBER, Ulrich, CH-8142 Uitikon-Waldegg (CH)
(74) Vertreter: Sika Patent Attorneys
(86) Internationale Anmeldenummer: PCT/EP2004/051519
(87) Internationale Veröffentlichungsnummer: WO 2005/007720

(56) Entgegenhaltungen:
- EP-A- 0 781 790
- WO-A-01/23466
- DE-A- 19 858 921
- GB-A- 1 326 669
- US-A- 3 533 983

## Beschreibung

### Technisches Gebiet

Die Erfindung betrifft hitzehärtende Zusammensetzungen, welche bei tiefen Temperaturen bis -40°C gleichzeitig eine hohe Schlagzähigkeit und gute mechanische Eigenschaften aufweisen und insbesondere als einkomponentige Klebstoffe eingesetzt werden können, sowie Schlagzähigkeitsmodifikatoren für Epoxidharze bei tiefen Temperaturen.

### Stand der Technik

In der Fertigung sowohl von Fahrzeugen und Anbauteilen oder auch Maschinen und Geräten werden anstelle oder in Kombination mit herkömmlichen Fügeverfahren wie Schrauben, Nieten, Stanzen oder Schweißen immer häufiger hochwertige Klebstoffe eingesetzt. Dadurch entstehen Vorteile und neue Möglichkeiten in der Fertigung, beispielsweise die Fertigung von Verbund- und Hybridwerkstoffen oder auch größere Freiheiten beim Design von Bauteilen. Die Klebstoffe müssen für eine Anwendung in der Fahrzeugherstellung gute Haftungen auf allen eingesetzten Untergründen, insbesondere elektrolytisch verzinkten, feuerverzinkten, und nachträglich phosphatierten Stahlblechen, beölten Stahlblechen sowie auf verschiedenen, gegebenenfalls oberflächenbehandelten, Aluminiumlegierungen aufweisen. Diese guten Haftungseigenschaften müssen besonders auch nach Alterung (Wechselklima, Salzsprühbad etc.) ohne grosse Qualitätseinbussen erhalten bleiben. Wenn die Klebstoffe als Rohbauklebstoffe im Automobilbau eingesetzt werden, ist die Beständigkeit dieser Klebstoffe gegenüber Reinigungsbädern und Tauchlackierung (sog. Auswaschbeständigkeit) von grosser Wichtigkeit, damit die Prozess-Sicherheit beim Hersteller garantiert werden kann.
Die Klebstoffe für den Rohbau müssen unter den üblichen Einbrennbedingungen von idealerweise 30 Min. bei 180°C aushärten. Des weiteren müssen sie aber auch bis circa 220°C beständig sein. Weitere Anforderungen für einen solchen gehärteten Klebstoff beziehungsweise der Verklebung sind die Gewährleistung der Betriebssicherheit sowohl bei hohen Temperaturen bis circa 90°C als auch bei tiefen Temperaturen bis circa -40°C. Da es sich bei diesen Klebstoffen um strukturelle Klebstoffe handelt und deshalb diese Klebstoffe strukturelle Teile verkleben, sind eine hohe Festigkeit und Schlagzähigkeit des Klebstoffes von grösster Wichtigkeit.

Herkömmliche Epoxidklebstoffe zeichnen sich zwar durch eine hohe mechanische Festigkeit, insbesondere eine hohe Zugfestigkeit aus. Bei schlagartiger Beanspruchung der Verklebung sind klassische Epoxidklebstoffe jedoch meist zu spröde und können deshalb unter Crashbedingungen, bei denen sowohl grosse Zug- als auch Schälbeanspruchungen auftreten, den Anforderungen, insbesondere der Automobilindustrie, bei weitem nicht genügen. Ungenügend sind diesbezüglich oft besonders die Festigkeiten bei hohen, insbesondere aber bei tiefen Temperaturen (< -10°C).

In der Literatur werden im Wesentlichen zwei Methoden vorgeschlagen, wie die Sprödigkeit von Epoxidklebstoffen reduziert und damit die Schlagzähigkeit erhöht werden kann: Einerseits kann das Ziel durch die Beimengung von zumindest teilvernetzten hochmolekularen Verbindungen wie Latices von Kern/Schale-Polymeren oder anderen flexibilisierenden Polymeren und Copolymeren erreicht werden. Andererseits kann auch durch Einführung von Weichsegmenten, z.B. durch die entsprechende Modifizierung der Epoxidkomponenten, eine gewisse Zähigkeitserhöhung erreicht werden.
Gemäss der erstgenannten Technik entsprechend der Lehre im Patent US 5,290,857 können Epoxidharze schlagzäher gemacht werden, indem ein feines, pulverförmiges Kern/Schalenpolymer in die Epoxidmatrix eingemischt wird. Dadurch entstehen in der hart-spröden Epoxidmatrix hochelastische Domänen, welche die Schlagzähigkeit erhöhen. Solche KemlSchalenpolymere sind in Patent US 5,290,857 basierend auf Acrylat- oder Methacrylat-Polymere beschrieben.
Gemäss der zweitgenannten Technik werden in Patent US 4,952,645 Epoxidharz-Zusammensetzungen beschrieben, welche durch die Umsetzung mit aliphatischen, cycloaliphatischen oder aromatischen Carbonsäuren, insbesondere di- oder trimeren Fettsäuren, sowie mit Carbonsäurenterminierten aliphatischen oder cyclo-aliphatischen Diolen flexibilisiert wurden. Solche Zusammensetzungen sollen sich durch eine erhöhte Flexibilität insbesondere bei tiefen Temperaturen auszeichnen.
EP 0 343 676 beschreibt einen reaktiven Hotmelt-Epoxidklebstoff mit einem Polyurethan-Epoxid-Addukt. Dabei werden die endständigen Isocyanatgruppen von Prepolymeren mit mindestens einem hydroxylgruppenhaltigen Epoxidharz einer OH-Funktionalität von grösser 2 derart umgesetzt, dass ein bei Raumtemperatur fester Schmelzklebstoff erhalten wird.
Bekannt ist auch, dass Epoxidharze mit reaktiven Elastomeren wie z.B. synthetischen Kautschuken und deren Derivaten flexibilisiert werden können. Der Haupteffekt betreffend die Zähelastifizierung beruht dabei auf der nur teilweisen Mischbarkeit der Epoxidharze und den entsprechenden derivatisierten synthetischen Kautschuken, wodurch beim Herstellprozess heterodisperse Phasen entstehen, welche einen den Kern/Schale-Polymeren vergleichbaren Effekt haben. Die Einstellung dieser Überstruktur ist jedoch sowohl von der mengenmässigen Zusammensetzung als auch von den Prozessführung während des Härtungsprozesses sehr abhängig. Dies führt dazu, dass eine kontinuierlich gleichbleibende Qualität sehr schwierig zu erreichen ist.
Als besonders vorteilhaft für die Schlagzähmodifizierung von Epoxidharzen werden in EP 0307666 A1 Elastomere mit Phenolendgruppen beschrieben, die durch Umsetzung von isocyanat-terminierten Prepolymeren mit einem hohen Überschuss an Bisphenolen hergestellt werden. Für die Formulierung mit Epoxiden nachteilig ist der hohe Phenolgehalt, der sich nachteilig auf die Lagerstabilität des formulierten Systems auswirken sowie bei der Aushärtung bei 180 °C zum Ausgasen führen kann.
Für die Heisshärtung der oben beschriebenen Epoxidharze werden meist latente Härter wie Dicyandiamid eingesetzt. Bekannt ist auch die Heisshärtung mittels Phenolhärtern wie Bisphenolen oder Novolacken. Sie führen zwar vorteilhaft gehärteten Klebstoffen mit hohen Glasübergangstemperaturen, stehen jedoch aufgrund von ökologischen Aspekten in der Diskussion.

### Darstellung der Erfindung

Es ist die Aufgabe der vorliegenden Erfindung, neue Schlagzähigkeitsmodifikatoren für Epoxidharz-Zusammensetzungen zur Verfügung zu stellen, die insbesondere kein freies Phenol enthalten und für den Einsatz bei tiefen Temperaturen, insbesondere auch Temperaturen von tiefer als-20°C, geeignet sind. Diese Schlagzähigkeitsmodifikatoren sollen vorzugsweise als Bestandteil von bei Raumtemperatur stabilen, einkomponentigen und hitzehärtenden Zusammensetzungen, insbesondere Klebstoffen und Schmelzklebstoffen, geeignet sein.
Überraschenderweise wurde gefunden, dass dies durch den Einsatz von Epoxidgruppen-terminierten polymeren Verbindungen der allgemeinen Formel (I) erreicht werden kann: 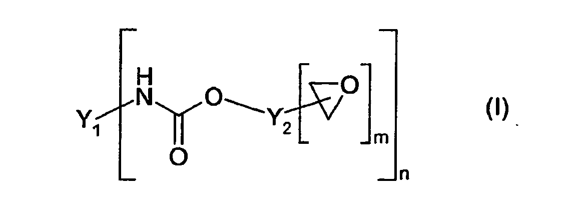 wobei Y₁ für einen n-wertigen Rest eines mit Isocyanatgruppen terminierten linearen oder verzweigten Polyurethanprepolymeren nach dem Entfemen der endständigen Isocyanatgruppen steht und Y₂ für einen Rest eines eine primäre oder sekundäre Hydroxylgruppe enthaltenden aliphatischen, cycloaliphatischen, aromatischen oder araliphatischen Epoxids nach dem Entfernen der Hydroxid- und Epoxidgruppen, steht, und n= 2, 3 oder 4 ist, sowie m = 1, 2 oder 3 ist. Das Polymer der Formel (I) weist zudem mindestens ein aromatisches Strukturelement auf, welches über Urethangruppen in der Polymerkette eingebunden ist.
Es hat sich gezeigt, dass dieses Polymer der Formel (I) einen guten Schlägzähigkeitsmodifikator darstellt.
Ein besonderer Aspekt der Erfindung stellt eine Zusammensetzung dar, welche mindestens ein Epoxid-Addukt **A** mit durchschnittlich mehr als einer Epoxidgruppe pro Molekül sowie mindestens ein Polymer **B** der Formel (I) sowie mindestens ein Thixotropiermittel **C,** auf Basis eines Harnstoffderivates in einem nicht-diffundierenden Trägermaterial, sowie mindestens einen Härter **D** für Epoxidharze, welcher durch erhöhte Temperatur aktiviert wird, umfasst.
Diese Zusammensetzung dient insbesondere als Klebstoff und weist einen ausserordentlich hohen Schlagschälarbeitswert, insbesondere bei tiefen Temperaturen, auf.
Gemäss bevorzugten Ausführungsformen sind weiterhin Zusammensetzungen beschrieben, die zusätzlich mindestens einen Füllstoff **E** und/oder mindestens einen Reaktiwerdünner **F** enthalten.
Weiterer Gegenstand der Erfindung sind Epoxidgruppen-terminierte Schlagzähigkeitsmodifikatoren der Formel (I). Es hat sich gezeigt, dass diese neuen Schlagzähigkeitsmodifikatoren eine bedeutende Schlagzähigkeitserhöhung in Epoxidharz-Zusammensetzungen, insbesondere 1-Komponentigen hitzehärtenden Epoxidharz-Zusammensetzungen sowie bei 2-Komponenten-Epoxidharz-Zusammensetzungen, bewirken.

### Weg zur Ausführung der Erfindung

Die vorliegende Erfindung betrifft Zusammensetzungen, welche mindestens ein Epoxid-Addukt **A** mit durchschnittlich mehr als einer Epoxidgruppe pro Molekül, mindestens ein Polymer **B** der Formel (I), mindestens ein Thixotropiermittel **C,** auf Basis eines Harnstoffderivates in einem nicht-diffundierenden Trägermaterial und mindestens einen Härter **D** für Epoxidharze, welcher durch erhöhte Temperatur aktiviert wird, enthalten.
Das Epoxid-Addukt **A** ist ein Epoxid-Addukt **A1** oder ein Epoxid-Addukt **A2.**
Das Epoxid-Addukt **A1** ist erhältlich aus der Reaktion von mindestens einer Dicarbonsäure und mindestens einem Diglycidylether. Das Epoxid-Addukt **A2** ist erhältlich aus der Reaktion von mindestens einem Bis(aminophenyl)sulfon-Isomeren oder von mindestens einem aromatischen Alkohol und mindestens einem Diglycidylether.
Die zur Herstellung des Epoxid-Addukt **A1** verwendete Dicarbonsäure ist vorzugsweise eine Dimerfettsäure. Als besonders geeignet gezeigt haben sich dimere C₄ - C₂₀ Fettsäuren, welche C₈- C₄₀ Dicarbonsäuren darstellen.
Bei den Diglycidylethern handelt es sich vorzugsweise um ein Flüssighatz, insbesondere Diglycidylether von Bisphenol-A (DGEBA), von Bisphenol-F sowie von Bisphenol-A/F (Die Bezeichnung 'A/F' verweist hierbei auf eine Mischung von Aceton mit Formaldehyd, welche als Edukt bei dessen Herstellung verwendet wird). Durch die Herstellungsverfahren dieser Harze bedingt, ist klar, dass in den Flüssigharzen auch höher molekulare Bestandteile enthalten sind. Solche Flüssigharze sind beispielsweise als Araldite GY 250, Araldite PY 304, Araldit GY 282 (Vantico) oder D.E.R 331 (Dow) erhältlich.
Das Epoxid-Addukt **A1** weist einen flexibilisierenden Charakter auf.

Das Epoxid-Addukt **A2** ist erhältlich durch die Reaktion mindestens eines Bis(aminophenyl)sulfon-Isomeren oder mindestens eines aromatischen Alkohols mit mindestens einem Diglycidylether. Der aromatische Alkohol ist bevorzugt ausgewählt aus der Gruppe von 2,2-Bis(4-hydroxyphenyl)propan (=Bisphenol-A), Bis(4-hydroxyphenyl)methan (=Bisphenol-F), Bis(4-hydroxyphenyl)sulfon (Bisphenol-S), Hydrochinon, Resorcin, Brenzkatechin, Naphthochinon, Naphtoresorcin, Dihydroxynaphthalin, Dihydroxyanthrachinon, Dihydroxy-biphenyl, 3,3-bis(p-hydroxyphenyl)phthalide, 5,5-Bis(4-hydroxyphenyl)hexahydro-4.,7-methanoindan, 4,4'-[bis-(hydroxyphenyl)-1,3-Phenylene-bis-(1-Methyl-ethyliden)] (=Bisphenol-M), 4,4'-[bis-(hydroxyphenyl)-1,4-Phenylenebis-(1-Methyl-ethyliden)] (=Bisphenol-P), sowie alle Isomeren der vorgenannten Verbindungen. Als besonders bevorzugter aromatischen Alkohol ist Bis(4-hydroxyphenyl)sulfon geeignet.
Die bevorzugten Bis(aminophenyl)sulfon-Isomeren sind Bis(4-aminophenyl)sulfon und Bis(3-aminophenyl)sulfon.
Die bevorzugten Diglycidylether sind die bereits für Epoxid-Addukt **A1** beschrieben Diglycidylether.
Das Epoxid-Addukt **A2** weist eine eher starre Struktur auf.

Besonders bevorzugt ist die gleichzeitige Anwesenheit von Epoxid-Addukt **A1** und Epoxid-Addukt **A2** in Zusammensetzungen gemäss Anspruch 1.
Das Epoxid-Addukt **A** weist bevorzugt ein Molekulargewicht von 700-6000 Dalton, vorzugsweise 900- 4000 Dalton, insbesondere 1000 - 3300 Dalton auf. Unter 'Molekulargewicht' oder 'Molgewicht` wird hier und im Folgenden das Molekulargewichtsmittel Mₙ verstanden.
Die Herstellung des Epoxid-Adduktes A erfolgt in der dem Fachmann bekannten Weise. Vorteilhaft wird am Ende der Adduktierung noch eine Zusatzmenge des oder der für die Adduktierung verwendeten Diglycidylether zugegeben und als Epoxid-Addukt **A**-Vormischung eingesetzt. In dieser Epoxid-Addukt **A**-Vormischung beträgt der Gesamtanteil des oder der nicht reagierten Diglycidylether 12-50 Gewichts-%, vorzugsweise 17-45 Gewichts-%, bezogen auf die Gewichtsumme der Epoxid-Addukt **A-**Vormischung.
Unter, Gesamtanteil' wird hier und im Folgenden jeweils die Summe aller zu dieser Kategorie gehörenden Bestandteile verstanden. Kommen beispielsweise in der Adduktierung gleichzeitig zwei verschiedene Diglycidylether vor, so ist als Gesamtanteil der Diglycidylether die Summe dieser zwei Diglycidylether zu verstehen.
Weiterhin vorteilhaft beträgt der Gewichtsanteil der Epoxid-Addukt **A-**Vormischung 20 - 70 Gewichts-%, vorzugsweise 35 - 65 Gewichts-%, bezogen auf das Gewicht der gesamten Zusammensetzung.

Das Polymer **B** ist durch Formel (I) darstellbar

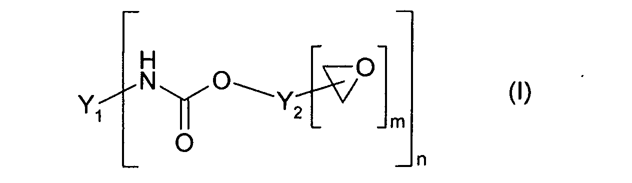

In Formel (I) stellt Y₁ einen n-wertigen Rest eines mit Isocyanatgruppen terminierten linearen oder verzweigten Polyurethanprepolymeren nach dem Entfernen der endständigen Isocyanatgruppen dar, und Y₂ steht für einen Rest eines eine primäre oder sekundäre Hydroxylgruppe enthaltenden aliphatischen, cycloaliphatischen, aromatischen oder araliphatischen Epoxids nach dem Entfernen der Hydroxid- und Epoxidgruppen. Des weiteren stehen die Indizes n für n = 2, 3 oder 4 und m für m = 1, 2 oder 3. Zudem weist das Polymer **B** mindestens ein aromatisches Strukturelement auf, welches über Urethangruppen in der Polymerkette eingebunden ist.
Das Polymer **B** der Formel (I) ist beispielsweise erhältlich durch die Reaktion einer Monohydroxy-Epoxidverbindung der Formel (II) und eines Isocyanatgruppen-terminierten linearen oder verzweigten Polyurethanprepolymeren der Formel (lll):

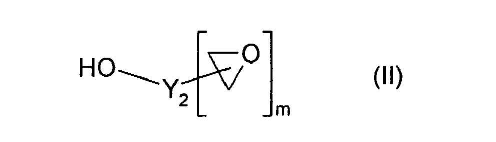

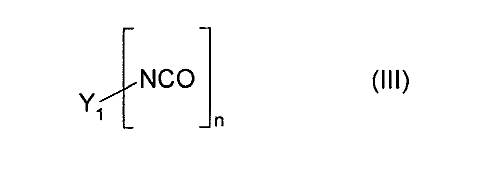

Zur Herstellung des Polyurethanprepolymers der Formel (III) werden mindestens ein Polyisocyanat, mindestens ein Polyphenol sowie mindestens ein Isocyanat-reaktives Polymer verwendet.

In der gesamten hier vorliegenden Schrift werden mit der Vorsilbe "Poly" in "Polyisocyanat", "Polyol", "Polyphenol" und "Polymerkaptan" Moleküle bezeichnet, die formal zwei oder mehr der jeweiligen funktionellen Gruppen enthalten.
Als Polyisocyanat sind geeignet Diisocyanate, Triisocyanate oder Tetraisocyanate, insbesondere Di- oder Triisocyanate. Bevorzugt sind Diisocyanate.
Als Diisocyanate sind geeignet aliphatische, cycloaliphatische, aromatische oder araliphatische Diisocyanate, insbesondere handelsübliche Produkte wie Methylendiphenyldiisocyanat (MDI), Hexamethylendiisocyanat (HDI), Toluoldiisocyanat (TDI), Tolidindiisocyanat (TODI), Isophorondiisocyanat (IPDI), Trimethylhexamethylendiisocyanat (TMDI), 2,5- oder 2,6-Bis-(isocyanatomethyl)- bicyclo[2.2.1]heptan, 9,5-Naphthalindüsocyanat (NDI), Dicyclohexylmethyldiisocyanat (H₁₂MDI), p-Phenylendiisocyanat (PPDI), m-Tetramethylxylylen diisocyanat (TMXDI), etc. sowie deren Dimere. Bevorzugt sind HDI, IPDI, TMDI , MDI, oder TDI .
Geeignete Triisocyanate sind insbesondere Trimere oder Biurete von aliphatischen, cycloaliphatischen, aromatischen oder araliphatischen Diisocyanaten, insbesondere die Isocyanurate und Biurete der im vorherigen Absatz beschriebenen Diisocyanate.

Als Polyphenole sind insbesondere geeignet Bis-, Tris- und Tetraphenole. Hierunter werden nicht nur reine Phenole, sondern gegebenenfalls auch substituierte Phenole verstanden. Die Art der Substitution kann sehr vielfältig sein. Insbesondere wird hierunter eine Substitution direkt am aromatischen Kern, an dem die phenolische OH-Gruppe hängt, verstanden. Unter Phenole werden weiterhin nicht nur einkernige Aromaten, sondern auch mehrkernige oder kondensierte Aromaten oder Heteroaromaten verstanden, welche die phenolische OH-Gruppe direkt am Aromaten beziehungsweise Heteroaromaten aufweisen.

Durch die Art und Stellung eines solchen Substituenten wird unter anderem die für die Bildung des Polyurethanprepolymeren der Formel (III) nötige Reaktion mit Isocyanaten beeinflusst.
Besonders eignen sich die Bis- und Trisphenole. Als Bisphenole oder Trisphenole sind beispielsweise geeignet 1,4-Dihydroxybenzol, 1,3-Dihydroxybenzol, 1,2-Dihydroxybenzol, 1,3-Dihydroxytoluol, 3,5-Dihydroxybenzoate, 2,2-Bis(4-hydroxyphenyl)propan (=Bisphenol-A), Bis(4-hydroxyphenyl)methan (=Bisphenol-F), Bis(4-hydroxyphenyl)sulfon (=Bisphenol-S), Naphtoresorcin, Dihydroxynaphthalin, Dihydroxyanthrachinon, Dihydroxy-biphenyl, 3,3-bis(p-hydroxyphenyl)phthalide, 5,5-Bis(4-hydroxyphenyl)hexahydro-4,7-methano-indan, Phenolpthalein, Fluorescein, 4,4'-[bis-(hydroxyphenyl)-1,3-Phenylene-bis-(1-Methyl-ethyliden)] (=Bisphenol-M), 4,4'-[bis-(hydroxyphenyl)-1,4-Phenylenebis-(1-Methyl-ethyliden)] (=Bisphenol-P), o,o-Diallyl-bisphenol-A, Diphenole und Dikresole hergestellt durch Umsetzung von Phenolen oder Kresolen mit Di-isopropylidenbenzol, Phloroglucin, Gallsäureester, Phenol-oder Kresolnovolacke mit -OH-Funktionalität von 2.0 bis 3.5 sowie alle Isomeren der vorgenannten Verbindungen.
Bevorzugte Diphenole und Dikresole hergestellt durch Umsetzung von Phenolen oder Kresolen mit Di-isopropylidenbenzol weisen eine chemische Strukturformel auf, wie sie entsprechend für Kresol als Bespiel nachfolgend gezeigt ist:

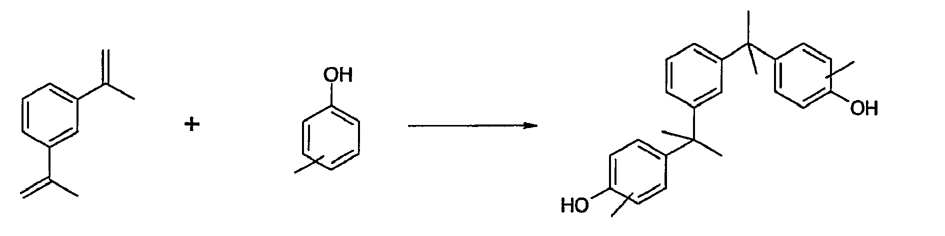

Besonders bevorzugt sind schwerflüchtige Bisphenole. Als meist bevorzugt gelten Bisphenol-M, Bisphenol-S.

Weiterhin wird für die Herstellung des Polyurethanprepolymers der Formel (III) mindestens ein Isocyanat-reaktives Polymer verwendet. Dieses Isocyanat-reaktive Polymer weist Isocyanat-reaktive Gruppen auf, die vorzugsweise Amino-, Thiol- oder Hydroxylgruppen, sind. Diese lsocyanat-reaktive Polymere weisen vorteilhaft ein Equivalenzgewicht von 600 - 6000, insbesondere von 600 - 4000, bevorzugt von 700 - 2200 g/Equivalent NCO-reaktiver Gruppen auf.
Insbesondere handelt es sich bei diesen Isocyanat-reaktiven Polymeren um Polyole, beispielsweise die folgenden handelsüblichen Polyole oder beliebige Mischungen davon:
- Polyoxyalkylenpolyole, auch Polyetherpolyole genannt, welche das Polymerisationsprodukt von Ethylenoxid, 1,2-Propylenoxid, 1,2- oder 2,3-Butylenoxid, Tetrahydrofuran oder Mischungen davon sind, gegebenenfalls polymerisiert mit Hilfe eines Startermoleküls mit zwei oder drei aktiven H-Atomen wie beispielsweise Wasser oder Verbindungen mit zwei oder drei OH-Gruppen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex Katalysatoren (kurz DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH oder Alkalialkoholaten. Speziell geeignet sind Polyoxypropylendiole und -triole mit einem Ungesättigtheitsgrad tiefer als 0.02 mEq/g und mit einem Molekulargewicht im Bereich von 1000 - 30'000 Dalton, Polyoxybutylendiole und -triole, Polyoxypropylendiole und -triole mit einem Molekulargewicht von 400 - 8'000 Dalton, sowie sogenannte "EO-endcapped" (ethylene oxide-endcapped) Polyoxypropylendiole oder -triole. Letztere sind spezielle Polyoxypropylenpolyoxyethylenpolyole, die beispielsweise dadurch erhalten werden, dass reine Polyoxypropylenpolyole nach Abschluss der Polypropoxylierung mit Ethylenoxid alkoxyliert werden und dadurch primäre Hydroxylgruppen aufweisen.
- Polyhydroxyterminierte Polybutadienpolyole, wie beispielsweise solche, die durch Polymerisation von 1,3-Butadien und Allylalkohol hergestellt werden;
- Styrol-Acrylnitril gepfropfte Polyetherpolyole, wie sie beispielsweise von Bayer unter dem Namen Lupranol geliefert werden;
- Polyhydroxyterminierte Acrylonitril/Polybutadien-Copolymere, wie sie beispielsweise aus Carboxylterminierten Acrylonitril/Polybutadien-Copolymere (kommerziell erhältlich unter dem Namen Hycar® CTBN von Hanse Chemie AG, Deutschland) und Epoxiden oder aus Aminoalkoholen hergestellt werden können;
- Polyesterpolyole, hergestellt beispielsweise aus zwei- bis dreiwertigen Alkoholen wie beispielsweise 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1 Trimethylolpropan oder Mischungen der vorgenannten Alkohole mit organischen Dicarbonsäuren oder deren Anhydride oder Ester wie beispielsweise Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure und Hexahydrophthalsäure oder Mischungen der vorgenannten Säuren, sowie Polyesterpolyole aus Lactonen wie beispielsweise ε-Caprolacton;
- Polycarbonatpolyole, wie sie durch Umsetzung beispielsweise der oben genannten - zum Aufbau der Polyesterpolyole eingesetzten- Alkohole mit Dialkylcarbonaten, Diarylcarbonaten oder Phosgen zugänglich sind.

Vorteilhaft sind die Isocyanat-reaktiven Polymere di- oder höherfunktioneller Polyole mit OH-Equivalentsgewichten von 600 bis 6000 g/OH-Equivalent, insbesondere von 600 bis 4000 g/OH-Equivalent, vorzugsweise 700 - 2200 g/OH-Equivalent. Weiterhin vorteilhaft sind die Polyole ausgewählt aus der Gruppe bestehend aus Polyethylenglycolen, Polypropylenglycolen, Polyethylenglycol-Polypropylenglycol-Block-Co-polymeren, Polybutylenglycolen, hydroxylterminierten Polybutadienen, hydroxylterminierten Polybutadien-co-Acrylnitrilen, hydroxylterminierten synthetischen Kautschuken und Gemischen dieser genannten Polyole.
Im Weiteren können als Isocyanat-reaktive Polymere auch mit di- oder höherfunktionellen aminterminierten Polyethylenethem, Polypropylenethern, Polybutylenethern, Polybutadienen, Polybutadien/Acrylnitrilen, wie sie zum Beispiel die unter dem Namen Hycar® CTBN von Hanse Chemie AG, Deutschland vertrieben werden, sowie weiteren aminterminierten synthetischen Kautschuken oder Gemischen der genannten Komponenten verwendet werden.
Es ist weiterhin möglich, dass Isocyanat-reaktive Polymere auch kettenverlängert sein können, wie sie gemäss dem Fachmann in bekannter Art und Weise aus der Reaktion von Polyaminen, Polyolen und Polyisocyanate, insbesondere aus Diamine, Diole und Diisocyanaten, hergestellt werden können.
Als Isocyanat-reaktive Polymere bevorzugt sind Polyole mit Molekulargewichten zwischen 600 und 6000 Dalton ausgewählt aus der Gruppe bestehend aus Polyethylenglykolen, Polypropylenglykolen, Polyethylenglykol-Polypropylenglykol-Blockpolymeren, Polybutylenglykolen, hydroxylterminierte Polybutadiene, hydroxylterminierte Polybutadien-Acrylnitril-Copolymere sowie deren Gemische.
Als lsocyanat-reaktive Polymere sind insbesondere bevorzugt α,ω-Polyalkylenglykole mit C₂-C₆-Alkylengruppen oder mit gemischten C₂-C₆-Alkylengruppen, die mit Amino-, Thiol- oder, bevorzugt, Hydroxylgruppen terminiert sind. Besonders bevorzugt sind Polypropylenglykol oder Polybutylenglykol.

Zur Herstellung des Polyurethanprepolymers der Formel (III) aus mindestens einem Polyisocyanat, mindestens einem Polyphenol sowie mindestens einem isocyanat-reaktiven Polymer stehen unterschiedliche Möglichkeiten zur Verfügung.
In einem ersten Verfahren, "Eintopfverfahren" genannt, wird eine Mischung von mindestens einem Polyphenol und mindestens einem Isocyanat-reaktiven Polymer mit mindestens einem Polyisocyanat in einem Isocyanatüberschuss umgesetzt.
In einem zweiten Verfahren, "2-Schrittverfahren I" genannt, wird mindestens ein Polyphenol mit mindestens einem Polyisocyanat in einem Isocyanatüberschuss umgesetzt und anschliessend mit mindestens einem Isocyanat-reaktiven Polymer in Unterschuss umgesetzt.
Im dritten Verfahren schliesslich, "2-chrittverfahren II" genannt, wird mit mindestens ein Isocyanat-reaktives Polymer mit einem Polyisocyanat in einem Isocyanatüberschuss umgesetzt und anschliessend mit mindestens einem Polyphenol in Unterschuss umgesetzt.
Die drei Verfahren führen zu Isocyanat-terminierten Polyurethanprepolymeren der Formel (III), die sich bei gleicher Zusammensetzung in der Sequenz ihrer Bausteine unterscheiden können. Es sind alle drei Verfahren geeignet, jedoch ist das "2-Schrittverfahren II" bevorzugt.

Werden die beschriebenen Isocyanat-endständigen Polymere der Formel (III) aus difunktionellen Komponenten aufgebaut, zeigte sich, dass das Equivalenz-Verhältnis lsocyanat-reaktives Polymer/Polyphenol bevorzugt grösser als 1.50 und das Equivalenz-Verhältnis Polyisocyanat/(Polyphenol + Isocyanat-reafctives Polymer) bevorzugt grösser als 1.20 ist.

Wird die durchschnittliche Funktionalität der verwendeten Komponenten grösser als 2, so erfolgt eine raschere Molekulargewichtserhöhung als im rein difunktionellen Fall. Für den Fachmann ist klar, dass die Grenzen der möglichen Equivalenz-Verhältnisse stark davon abhängen, ob entweder das gewählte lsocyanat-reaktive Polymer, das Polyphenol, das Polyisocyanat oder mehrere der genannten Komponenten eine Funktionalität >2 besitzen. Je nach dem können unterschiedliche Equivalenz-Verhältnisse eingestellt werden, deren Grenzen durch die Viskosität der resultierenden Polymere bestimmt wird und die experimentell von Fall zu Fall bestimmt werden müssen.

Das Polyurethanprepolymer der Formel (III) weist bevorzugt elastischen Charakter auf und zeigt eine Glasumwandlungstemperatur Tg von kleiner als 0°C.
Die Monohydroxy-Epoxidverbindung der Formel (II) weist 1, 2 oder 3 Epoxidgruppen auf. Die Hydroxylgruppe dieser Monohydroxy-Epoxidverbindung (II) kann eine primäre oder eine sekundäre Hydroxylgruppe darstellen.
Solche Monohydroxy-Epoxidverbindungen lassen sich beispielsweise durch Umsetzung von Polyolen mit Epichlorhydrin erzeugen. Je nach Reaktionsführung entstehen bei der Umsetzung von mehrfunktionellen Alkoholen mit Epichlorhydrin als Nebenprodukte auch die entsprechenden Monohydroxy-Epoxidverbindungen in unterschiedlichen Konzentrationen. Diese lassen sich durch übliche Trennoperationen isolieren. In der Regel genügt es aber, das bei der Glycidylisierungsreaktion von Polyolen erhaltene Produktgemisch aus vollständig und partiell zum Glycidylether reagiertem Polyol einzusetzen. Beispiele solcher hydroxylhaltigen Epoxide sind Trimethylolpropandiglycidylether (als Gemisch enthalten in Trimethylolpropantriglycidylether), Glycerindiglycidylether (als Gemisch enthalten in Glycerintriglycidylether), Pentaerythrittriglycidylether (als Gemisch enthalten in Pentaerythrittetraglycidylether). Vorzugsweise wird Trimethylolpropandiglycidylether, welcher zu einem relativ hohen Anteil in üblich hergestellten Trimethylolpropantriglycidylether vorkommt, verwendet.
Es können aber auch andere ähnliche hydroxylhaltige Epoxide, insbesondere Glycidol, 3-Glycidyloxybenzylalkohol oder Hydroxymethyl-cyclohexenoxid eingesetzt werden. Weiterhin bevorzugt ist der β-Hydroxyether der Formel (VI), der in handelsüblichen flüssigen Epoxidharzen hergestellt aus Bisphenol-A (R = CH₃) und Epichlorhydrin zu etwa 15 % enthalten ist, sowie die entsprechenden β-Hydroxyether (VI), die bei der Reaktion von Bisphenol-F (R = H) oder des Gemisches von Bisphenol-A und Bisphenol-F mit Epichlorhydrin gebildet werden.

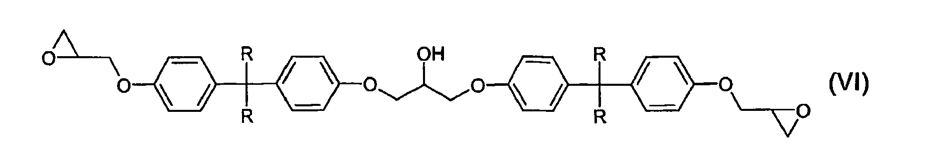

Im Weiteren können auch unterschiedlichste Epoxide mit einer β-Hydroxyether-Gruppe, hergestellt durch die Reaktion von (Poly-)Epoxiden mit einem Unterschuss von einwertigen Nukleophilen wie Carbonsäuren, Phenolen, Thiolen oder sec.- Aminen, eingesetzt werden.

Die freie primäre oder sekundäre OH-Funktionalität der Monohydroxy-Epoxidverbindung der Formel (II) lässt eine effiziente Umsetzung mit terminalen Isocyanatgruppen von Prepolymeren zu, ohne dafür unverhältnismässige Überschüsse der Epoxidkomponente einsetzen zu müssen.

Zur Umsetzung der Polyurethanprepolymeren der Formel (III) können stöchiometrische Mengen Monohydroxy-Epoxidverbindung der Formel (II) oder ihrer Mischungen eingesetzt werden. Von der Stöchiometrie in Bezug auf dessen Equivalente OH-Gruppen respektive Isocyanatgruppen kann abgewichen werden. Das Verhältnis [OH]/[NCO] beträgt 0.6 bis 3.0, vorzugsweise 0.9 bis 1.5, insbesondere 0.98 bis 1.1.

Das Polymer B weist mindestens ein aromatisches Strukturelement auf, welches über Urethangruppen in der Polymerkette eingebunden ist. Dieses Strukturelement lässt sich durch Formel (IV) veranschaulichen. Weiterhin ist gleichzeitig in der Polymerkette des Polymers B ein zweites Strukturelement vorhanden, welches sich durch Formel (V) veranschaulichen lässt:

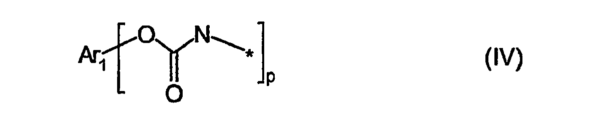

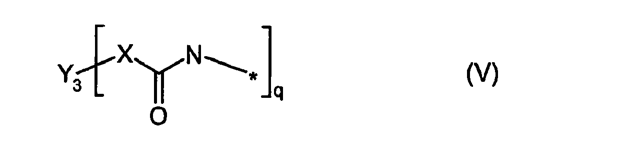

Der Index p steht für die Werte 2, 3 oder 4, insbesondere p = 2 oder 3, während der Index q die Werte 2, 3 oder 4, insbesondere q = 2 oder 3, darstellt. Weiterhin steht X für S, O oder NH, insbesondere für O. Der Rest Ar₁ stellt einen p-valenten, gegebenenfalls substituierten, Arylrest, dar. Der Rest Y₃ stellt einen q-wertigen Rest eines, gegebenenfalls kettenverlängerten, Isocyanat-reaktiven Polymers nach dem Entfemen der endständigen Amino-, Thiol- oder Hydroxylgruppen dar. Schliesslich stellt * in Formel (IV) und (V) die Verbindung die Anbindungsstelle zum Rest der Polymerkette dar. Diese Strukturelemente resultieren durch die bereits beschriebenen Reaktionen zur Herstellung des Polymeren **B.**

Das Polymer **B** weist vorteilhaft einen elastischen Charakter auf und ist weiterhin vorteilhaft in Epoxidharzen löslich oder dispergierbar.

Das Polymer **B** kann bei Bedarf und je nach der resultierenden Viskosität mit weiteren Epoxidharzen verdünnt werden. Bevorzugt sind hierzu Diglycidylether von Bisphenol-A, Bisphenol-F sowie von Bisphenol-A/F, sowie die weiter unten beschriebenen epoxidgruppentragenden Reaktiwerdünner **F,** insbesondere Hexandioldiglycidylether, Polypropylenglykoldiglycidylether und Trimethylolpropantriglycidylether.

Vorteilhaft beträgt der Gesamtanteil des Polymeren **B** 5 - 40 Gewichts-%, vorzugsweise 7 - 35 Gewichts-%, bezogen auf das Gewicht der gesamten Zusammensetzung.

Weiterhin enthält die Zusammensetzung mindestens ein Thixotropiermittel **C,** auf Basis eines Harnstoffderivates in einem nicht-diffundierenden Trägermaterial. Die Herstellung von solchen Harnstoffderivaten und Trägermaterialien sind im Detail in der Patentanmeldung EP 1 152 019 A1 beschrieben. Das Trägermaterial ist vorteilhaft ein blockiertes Polyurethanprepolymer **C1,** insbesondere erhalten durch Umsetzung eines trifunktionellen Polyetherpolyols mit IPDI und anschliessender Blockierung der endständigen Isocyanatgruppen mit Caprolactam.
Das Harnstoffderivat ist ein Umsetzungsprodukt eines aromatischen monomeren Diisocyanates mit einer aliphatischen Aminverbindung. Es ist auch durchaus möglich, mehrere unterschiedliche monomere Diisocyanate mit einer oder mehreren aliphatischen Aminverbindungen oder ein monomeres Diisocyanat mit mehreren aliphatischen Aminverbindungen umzusetzen. Als besonders vorteilhaft hat sich das Umsetzungsprodukt von 4,4'-Diphenyl-methylen-diisocyanat (MDI) mit Butylamin erwiesen.
Vorteilhaft beträgt der Gesamtanteil des Thixotropiermittels **C** 5 - 40 Gewichts-%, vorzugsweise 7-25 Gewichts-%, bezogen auf das Gewicht der gesamten Zusammensetzung. Der Anteil des Harnstoffderivats beträgt vorteilhaft 5 - 50 Gewichts-%, vorzugsweise 15 - 30 Gewichts-%, bezogen auf das Gewicht des Thixotropiermittels **C.**

Die erfindungsgemässe Zusammensetzung enthält weiterhin mindestens einen Härter D für Epoxidharze, welcher durch erhöhte Temperatur aktiviert wird. Es handelt sich hierbei vorzugsweise um einen Härter, welcher ausgewählt ist aus der Gruppe Dicyandiamid, Guanamine, Guanidine, Aminoguanidine und deren Derivate. Weiterhin möglich sind katalytisch wirksame substituierte Harnstoffe wie 3-Chlor-4-Methylphenylhamstoff (Chlortoluron), oder Phenyl-Dimethylharnstoffe, insbesondere p-Chlorphenyl-N,N-dimethylharnstoff (Monuron), 3-Phenyl-1,1-dimethylharnstoff (Fenuron) oder 3,4-Dichlorphenyl-N,N-dimethylharnstoff (Diuron). Weiterhin können Verbindungen der Klasse der Imidazole und Amin-Komplexe eingesetzt werden. Besonders bevorzugt ist Dicyandiamid.

Vorteilhaft beträgt der Gesamtanteil des Härters **D** 1 - 10 Gewichts-%, vorzugsweise 2-8 Gewichts-%, bezogen auf das Gewicht der gesamten Zusammensetzung.

In einer bevorzugten Ausführungsform enthält die Zusammensetzung zusätzlich mindestens einen Füllstoff **E.** Bevorzugt handelt es sich hierbei um Glimmer, Talk, Kaolin, Wollastonit, Feldspat, Chlorit, Bentonit, Montmorillonit, Calciumcarbonat (gefällt oder gemahlen), Dolomit, Quarz, Kieselsäuren (pyrogen oder gefällt), Cristobalit, Calciumoxid, Aluminiumhydroxid, Magnesiumoxid, Keramikhohlkugeln, Glashohlkugeln, organische Hohlkugeln, Glaskugeln, Farbpigmente. Als Füllstoff **E** sind sowohl die organisch beschichteten als auch die unbeschichteten kommerziell erhältlichen und dem Fachmann bekannten Formen gemeint.
Vorteilhaft beträgt der Gesamtanteil des gesamten Füllstoffs **E** 5 - 30 Gewichts-%, vorzugsweise 10-25 Gewichts-%, bezogen auf das Gewicht der gesamten Zusammensetzung.

In einer weiteren bevorzugten Ausführungsform enthält die Zusammensetzung zusätzlich mindestens einen epoxidgruppentragenden Reaktiwerdünner **F.** Bei diesen Reaktiwerdünnern **F** handelt es sich insbesondere um:
- Glycidylether von monofunktionellen gesättigten oder ungesättigten, verzweigten oder unverzweigten, zyklischen oder offenkettigen C₄- C₃₀ Alkoholen, z.B. Butanolglycidylether, Hexanolglycidylether, 2-Ethylhexanolether, Allylglycidylether, Tetrahydrofurfuryl- und Furfurylglycidylether, Trimethoxysilylglycidylether etc.
- Glycidylether von difunktionellen gesättigten oder ungesättigten, verzweigten oder unverzweigten, zyklischen oder offenkettigen C₂- C₃₀ Alkolen, z.B Aethylenglykol-, Butandiol-, Hexandiol-, Oktandiolgylcidylether, Cyclohexandimethanoldigylcidylether, Neopentylglycoldiglycidylether etc.
- Glycidylether von tri- oder polyfunktionellen, gesättigten oder ungesättigten, verzweigten oder unverzweigten, zyklischen oder offenkettigen Akoholen wie epoxidiertes Rhizinusöl, epoxidiertes Trimethylolpropan, epoxidiertes Pentaerythrol oder Polyglycidylether von aliphatischen Polyolen wie Sorbitol, Glycerin, Trimethylolpropan etc.
- Glycidylether von Phenol- und Anilinverbindungen wie Phenylglycidylether, Kresolglycidylether, p-tert.-Butylphenylglycidylether, Nonylphenolglycidylether, 3-n-Pentadecenyl-glycidylether (aus Cashewnuss-Schalen-ÖI), N,N-Diglycidylanilin etc.
- Epoxidierte Tertiäre Amine wie N, N-Diglycidylcyclohexylamin etc.
- Epoxidierte Mono- oder Dicarbonsäuren wie Neodecansäureglycidylester, Methacrylsäureglycidylester, Benzoesäureglycidylester, Phthalsäure-, Tetra- und Hexahydrophthalsäurediglycidylester, Diglycidylester von dimeren Fettsäuren etc.
- Epoxidierte di- oder trifunktionelle, nieder- bis hochmolekulare Polyetherpolyole wie Polyethylenglycol-diglycidylether, Polypropyleneglycoldiglycidylether etc.

Besonders bevorzugt sind Hexandioldiglycidylether, Polypropylenglycoldiglycidylether und Polyethylenglycoldiglycidylether.

Vorteilhaft beträgt der Gesamtanteil des epoxidgruppentragenden Reaktiwerdünners **F** 1 - 7 Gewichts-%, vorzugsweise 2-6 Gewichts-%, bezogen auf das Gewicht der gesamten Zusammensetzung.

Es hat sich gezeigt, dass sich die erfindungsgemässe Zusammensetzung besonders als einkomponentige Klebstoffe eignen. Insbesondere sind hiermit hitzehärtende einkomponentige Klebstoffe realisierbar, die sich durch eine hohe Schlagzähigkeit sowohl bei höheren Temperaturen und vor allem bei tiefen Temperaturen, insbesondere zwischen 0°C bis -40°C auszeichnen. Solche Klebstoffe werden für das Verkleben von hitzestabilen Materialien benötigt. Unter hitzestabilen Materialien werden Materialien verstanden, welche bei einer Aushärtetemperatur von 100 - 220 °C, vorzugsweise 120 - 200°C zumindest während der Aushärtezeit formstabil sind. Insbesondere handelt es sich hierbei um Metalle und Kunststoffe wie ABS, Polyamid, Polyphenylenether, Verbundmaterialien wie SMC, ungesättigte Polyester GFK, Epoxid- oder Acrylatverbundwerkstoffe. Bevorzugt ist die Anwendung, bei der zumindest ein Material ein Metall ist. Als besonders bevorzugte Verwendung gilt das Verkleben von gleichen oder verschiedenen Metallen, insbesondere im Rohbau in der Automobilindustrie. Die bevorzugten Metalle sind vor allem Stahl insbesondere elektrolytisch verzinkter, feuerverzinkter, beölter Stahl, Bonazink-beschichteter Stahl, und nachträglich phosphatierter Stahl, sowie Aluminium insbesondere in den im Autobau typischerweise vorkommenden Varianten.

Mit einem Klebstoff basierend auf einer erfindungsgemässen Zusammensetzung kann vor allem die gewünschte Kombination von hoher Crashfestigkeit und hoher sowie tiefer Einsatztemperatur erreicht werden.
Ein solcher Klebstoff wird zuerst mit den zu verklebenden Materialien bei einer Temperatur von zwischen 10° C und 80°C, insbesondere zwischen 10°C und 60°C, kontaktiert und später ausgehärtet bei einer Temperatur von typischerweise 100-220°C, vorzugsweise 120 - 200°C.

Selbstverständlich können mit einer erfindungsgemässen Zusammensetzung neben hitzehärtenden Klebstoffen auch Dichtmassen oder Beschichtungen realisiert werden. Ferner eignen sich die erfindungsgemässen Zusammensetzungen nicht nur für den Automobilbau sondern auch für andere Anwendungsgebiete. Besonders naheliegend sind verwandte Anwendungen in Transportmittelbau wie Schiffe, Lastwagen, Busse oder Schienenfahrzeuge oder im Bau von Gebrauchsgütern wie beispielsweise Waschmaschinen.

Die mittels einer erfindungsgemässen Zusammensetzung verklebten Materialien kommen bei Temperaturen zwischen typischerweise 100°C und -40°C, vorzugsweise zwischen 80°C und -40°C, insbesondere zwischen 50°C und -40°C zum Einsatz.

Die Zusammensetzungen weisen typischerweise Bruchenergie, gemessen nach DIN 11343, von mehr als 10.0 J bei -20°C und mehr als 7.0 J bei -40°C auf. Bevorzugt sind Bruchenergien von mehr als 11.0 J bei -20°C und von mehr als 9.0 J bei -40°C.

In einer speziellen Weise sind auch Schmelzklebstoffe auf der Basis der erfindungsgemässen Zusammensetzung realisierbar. Hierbei werden zusätzlich die beim Epoxid-Addukt **A** entstehenden Hydroxygruppen mit Polyisocyanat, beziehungsweise einem Polyisocyanat-Prepolymer, umgesetzt. Dadurch wird die Viskosität erhöht, und eine Warmapplikation erforderlich.

Ein weiterer Aspekt der Erfindung sind neue Epoxidgruppenterminierter Schlagzähigkeitsmodifikatoren der Formel (I) des Polymeren **B,** deren detaillierte Konstitution und Wege zur Herstellung bereits weiter vorne beschrieben worden sind.
Es hat sich gezeigt, dass diese Epoxidgruppen-terminierten Schlagzähigkeitsmodifikatoren der Formel (I) Epoxidharz-haltigen Zusammensetzungen beigefügt werden können. Es sind Systeme möglich, welche auch ohne Addukte formuliert werden können. Es sind sowohl einkomponentige als auch zwei- oder mehrkomponentige Systeme möglich, die raumtemperaturhärtend oder hitzehärtend sein können. Neben den bereits beschriebenen hitzehärtenden 1-Komponenten Zusammensetzungen eignen sie sich auch gut bei 2- oder Mehr-Komponenten Epoxidharzzusammensetzungen, insbesondere für solche, deren zweite Komponente ein Amin- beziehungsweise ein Polyaminhärter oder ein Merkaptan- beziehungsweise eine Polymerkaptanhärter darstellt. Die Epoxidgruppen-terminierte Schlagzähigkeitsmodifikatoren der Formel (I) werden der Härterkomponente zugegeben, wobei ein oder mehrere Addukte gebildet werden, oder, vorzugsweise, derjenigen Komponente zugegeben, welche das Epoxidharz enthält. Weitere, weniger bevorzugte, Möglichkeiten sind die Zugabe eines Epoxidgruppen-terminierten Schlagzähigkeitsmodifikators direkt bei der Applikation oder die Zugabe als Bestandteil einer dritten oder weiteren Komponente bei der Applikation.
Die Aushärtungstemperatur solcher 2- oder Mehr-Komponenten-Epoxidharzzusammensetzungen ist vorzugsweise zwischen 10°C und 60°C, insbesondere zwischen 15°C und 50°C. Epoxidgruppen-terminierte Schlagzähigkeitsmodifikatoren der Formel (I) eignen sich insbesondere als Zusatz zu 2-Komponenten Epoxidharzklebstoffen. Die Erhöhung der Schlagzähigkeit ist hierbei nicht auf tiefe Temperaturen beschränkt.
Von besonderem Interesse ist die partielle Vorhärtung der erfindungsgemässen Epoxidgruppen-terminierten Schlagzähigkeitsmodifikatoren durch Polyamine oder Polymerkaptane, insbesondere durch Diamine und Dimerkaptane. Dadurch kann das 2-Komponentige System so eingestellt werden, dass der Klebstoff durch eine partielle Vorvemetzung eine hoch viskose bis gummiartige Konsistenz erhält, welche die Auswaschbeständigkeit in Waschprozessen bei Temperaturen bis 70°C gewährleistet.
Diese Zusammensetzungen, insbesondere Klebstoffe, werden unmittelbar vor der Applikation mit einem 2- oder Mehr-Komponentenmischgerät auf die zu kontaktierenden Materialien aufgetragen. Solche 2- oder Mehrkomponentenklebstoffe können sowohl im Automobilbau als auch im Transportmittelbau (Schiffe, Lastwagen, Busse oder Schienenfahrzeuge) oder im Bau von Gebrauchsgütern wie beispielsweise Waschmaschinen, aber auch im Bausektor beispielsweise als versteifende Strukturklebstoffe (u.a. Verbundwerkstoffe etc.) eingesetzt werden.
Ein solcher zweikomponentiger Klebstoff kann beispielsweise derart formuliert sein, dass der Schlagzähigkeitsmodifikators Bestandteil der ersten Komponente ist und mindestens ein Polyamin oder mindestens ein Polymerkaptan Bestandteil der zweiten Komponente ist.
Solche Klebstoffe weisen nach dem Mischen und Aushärten ebenfalls gute Haftung auf den bereits oben beschriebenen Untergründen auf.

### Beispiele

Im Folgenden sollen einige Beispiele aufgezeigt werden, welche die Erfindung weiter veranschaulichen, den Umfang der Erfindung aber in keiner Weise beschränken sollen. Die in den Beispielen verwendeten Rohstoffe sind in Tabelle1 aufgelistet.

**Tabelle1. Eingesetzte Rohstoffe.**

| **Verwendete Rohstoffe** | **Lieferant** |
|---|---|
| Dimerisierte C18-Fettsäure (Pripol 1013) | Uniqema |
| Triphenylphosphin | Fluka AG |
| Bis(4-hydroxyphenyl)sul fon (= Bisphenol-S) | Fluka AG |
| Bisphenol-A-diglycidylether (= DGEBA) | Vantico |
| Polypropylenglycol-diglycidylether (ED-506) | Asahi-Denka Kogyo |
| Dicyandiamid (= Dicy) | Degussa |
| Isophoron-diisocyanat (= IPDI). | Degussa-Hüls |
| Caprolactam | EMS Chemie |
| N-Butylamin | BASF |
| 4,4'-Diphenyl-methylen-düsocyanat (= MDI) | Bayer |
| 2,4-Trimethylhexamethylen-1,6-diisocyanat (= TMDI) | Degussa-Hüls |
| Hexandioldiglycidylether | Prümmer |
| Desmophen 3060 BS (Trifunktionelles Polypropylenglykol, OH-Equivalentgewicht = 1000 g/OH-Equivalent) | Bayer |
| PolyTHF 2000 / PolyTHF2900 (Difunktionelles Polybutylen-glykol, OH-Equivalentgewicht = 1000 bzw. 1450 g/OH-eq.) | BASF |
| Liquiflex P (Hydroxylterminertes Polybutadien, OH-Equivalentgewicht = ca. 1200 g/OH-Equivalent ) | Petroflex |
| Priplast 2033 (hydroxyterminierte dimerisierte C18-Fettsäure) | Uniqema |
| Bis-(hydroxymethyl)tricyclo[5.2.1.0(2.6)]decan (= TCD-DM) | Aldrich |
| 4,4'-Isopropyliden-dicyclohexanol(= hydriertes Bisphenol-A,=A/H) | Aldrich |
| Bisphenol-A | Fluka AG |
| 4,4'-[bis-(hydroxyphenyl)-1,3-Phenylene-bis-(1-Methyl-ethyliden)] (=Bisphenol-M) | Mitsui Chemicals |
| Resorcin | Fluka AG |
| Phenolphthalein | Fluka AG |
| o-Kresol | Fluka AG |
| Lewatit 1131 (anionisches Ionentauscherharz) | BASF |
| 1,3-Diisopropenyl-benzol (= m-DIPEP) | Cytec |

Allgemeine Herstellung des Epoxidadduktes **A,** beziehungsweise der Epoxid-Addukt **A**-Vormischung:

### Beispiel für Epoxid-Addukt A-Vormischung: A-VM1

Bei 110°C wurden unter Vakuum und Rühren 123.9 g einer dimeren Fettsäure, 1.1 g Triphenylphosphin sowie 71.3 g Bis(4-hydroxyphenyl)sulfon mit 658 g eines flüssigen DGEBA-Epoxidharzes mit einem Epoxidgehalt von 5.45 eq/kg 5 Stunden lang umgesetzt, bis eine konstante Epoxidkonzentration von 2.82 eq/kg erreicht war. Nach dem Ende der Reaktion wurden dem Reaktionsgemisch **A** zusätzlich 187.0 g flüssigen DGEBA-Epoxidharzes zugegeben.

### Beispielhafte Herstellung eines monohydroxylhaltigen Epoxides

Trimethylolpropanglycidylether wurde gemäss dem Verfahren in Patent US 5,668,227, Beispiel 1 aus Trimethylolpropan und Epichlorhydrin mit Tetramethylammoniumchlorid und Natronlauge hergestellt. Man erhält ein gelbliches Produkt mit Epoxidzahl von 7.5 eq/kg und einem Hydroxylgruppengehalt von 1.8 eq/kg. Aus dem HPLC-MS Spektrum kann geschlossen werden, dass im Wesentlichen ein Gemisch von Trimethylolpropandiglycidylether und Trimethylolpropantriglycidylether vorliegt.

### Beispielhafte Herstellung eines Bisphenols (Bis-OK5)

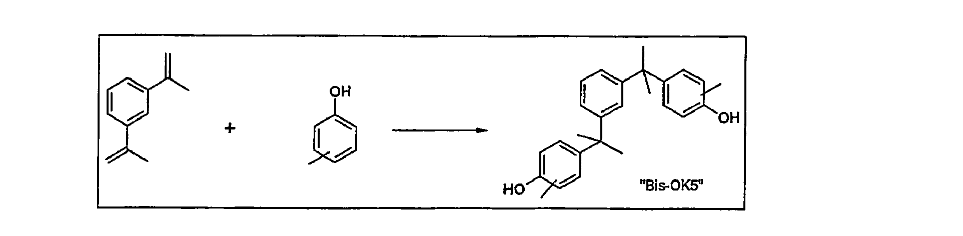

864 g (8.0 mol) o-Kresol und 100 g Lewatit-1131 (Katalysator) wurden bei einem Druck von 0.05 bar auf 67°C erwärmt und es wurden 50 ml H₂O (ex Lewatit-1131) abdestilliert. Anschliessend wurde unter N₂-Atmosphäre während 1 h 316 g (2.0 mol) m-DIPEP zugetropft, wobei die Temperatur langsam auf 105°C anstieg. Es wurde für 3 h bei 95°C unter N₂-Atmosphäre gerührt. Danach wurde der Katalysator über ein Drahtnetz abfiltriert. Bei einem Druck von 0.05 bar wurde anschliessend die Temperatur während 1 h schrittweise auf 230°C erhöht, wobei insgesamt 500 ml o-Kresol abdestilliert werden konnten. Auf diese Weise wurden 680 g einer hochviskosen, honiggelben Masse mit einem Restmonomergehalt von 0.58 % und einem OH-Gehalt von ca. 5 eq/kg erhalten.

Im Folgenden werden unterschiedliche Beispiele der Herstellung des Polymers **B** der Formel (I) gezeigt.

### Beispiel eines Polymers B (erfindundsgemäss): B-01

200 g PolyTHF 2000 (OH-Zahl 57.5 mg/g KOH) wurden 30 Minuten unter Vakuum bei 100°C getrocknet. Anschliessend wurden 47.5 g IPDI und 0.04 g Dibutylzinndilaurat zugegeben. Die Reaktion wurde unter Vakuum bei 90°C bis zur Konstanz des NCO-Gehaltes bei 3.6% nach 2.5 h geführt (theoretischer NCO-Gehalt: 3.7%). Danach wurden 17.7 g Bisphenol-M zugegeben (Verhältnis NCO / OH : 0.45) und es wurde unter Vakuum bei 90°C wiederum bis zur Konstanz des NCO-Gehaltes bei 2.1 % nach 3 h weitergerührt (theoretischer NCO-Gehalt: 2.0%). Anschliessend wurden 78.1 g des oben beschriebenen Trimethylolpropanglycidylethers als monohydroxylhaltiges Epoxid der Formel (II) zugegeben. Es wurde bei 90°C unter Vakuum weitergerührt, bis der NCO-Gehalt nach weiteren 3h unter 0.1 % gesunken war. Nach Abschluss der Reaktion wurden 82.9 g DGEBA zugegeben (1/3 der Masse des unblockierten, NCO-endständigen Prepolymers). So wurde ein klares Produkt mit einem Epoxid-Gehalt ("End-EP-Gehalt") von 2.51 eq/kg erhalten.

### Weitere Polymere B (erfindungsgemäss): B-02 bis B-09

Tabelle 2 weist weitere Beispiele für Polymere, wie sie in erfindungsgemässen Zusammensetzungen zum Einsatz kommen. Diese Polymere werden in gleicher Weise wie Beispiel *B-01* hergestellt.

### Beispiel eines Polymers P (nicht erfindunasaemäss): P-01

200 g PolyTHF 2000 (OH-Zahl 57.5 mg/g KOH) wurden 30 Minuten unter Vakuum bei 100°C getrocknet. Anschliessend wurden 47.5 g IPDI und 0.04 g Dibutylzinndilaurat zugegeben. Die Reaktion wurde unter Vakuum bei 90°C bis zur Konstanz des NCO-Gehaltes bei 3.6% nach 2.5 h geführt (theoretischer NCO-Gehalt: 3.7%). Anschliessend wurden 123.7 g des oben beschriebenen Trimethylolpropanglycidylethers als monohydroxylhaltiges Epoxid der Formel (II) zugegeben. Es wurde bei 90°C unter Vakuum weitergerührt, bis der NCO-Gehalt nach weiteren 3h unter 0.1% gesunken war. Nach Abschluss der Reaktion wurden 82.5 g DGEBA zugegeben (1/3 der Masse des unblockierten, NCO-endständigen Prepolymers). So wurde ein klares Produkt mit einem Epoxid-Gehalt ("End-EP-Gehalt") von 3.15 eq/kg erhalten.

### P-01 enthält also keine Polyphenol-Struktureinheiten in der Polymerkette.

### Weitere Beispiele Polymere P (nicht erfindungsgemäss): P-02 bis P-05

Die Polymere *P-02* bis *P-05* werden nach Tabelle 2 in gleicher Weise wie das Polymer *P-01* beziehungsweise analog zu ***B-01*** hergestellt. Bei Polymer ***P-02*** wurde die gleiche Menge Bisphenol-M wie in Beispiel *B-01* verwendet, allerdings wurde Bisphenol-M es ganz am Schluss der Synthese im heissen Polymer gelöst. Das Polymer ***P-02*** enthält demzufolge freies, ungebundenes Bisphenol-M. Die Polymeren *P-03, P-04* und ***P-05*** enthalten anstelle des Bisphenols-M des Beispiels ***B-01*** aliphatische Diole.

**Tabelle 2. Polymere**

| | | ***B-01*** | ***B-02*** | ***B-03*** | ***B-04*** | ***B-05*** | ***B-06*** | ***B-07*** | ***B-08*** | ***B-09*** | ***P-01*** | ***P-02*** | ***P-03*** | ***P-04*** | ***P-05*** |
|---|---|---|---|---|---|---|---|---|---|---|---|---|---|---|---|
| Polyole | OH-Zahl [mg/g KOH] | [g] | [g] | [g] | [g] | [g] | [g] | [g] | [g] | [g] | [g] | [g] | [g] | [g] | [g] |
| pTHF2000 | 57.5 | 200.0 | 200.0 | 200.0 | 200.0 | 200.0 | 200.0 | | | 160.0 | 200.0 | 200.0 | 200.0 | 200.0 | 200.0 |
| pTHF2900 | 40.6 | | | | | | | 200.0 | 200.0 | | | | | | |
| Liquiflex-P | 45.0 | | | | | | | | | 40.0 | | | | | |
| Polyisocyanate | Equiv.Gewicht [g/eq] | | | | | | | | | | | | | | |
| IPDI | 111.2 | 47.5 | 47.5 | 47.5 | 47.5 | | 47.5 | 33.9 | 33.9 | 46.8 | 47.5 | 47.5 | 47.5 | | 47.5 |
| TMDI | 105.0 | | | | | 45.9 | | | | | | | | 45.9 | |
| Polyphenole | Equiv.Gewicht [g/eq] | | | | | | | | | | | | | | |
| Bisphenol-M | 173.0 | 17.7 | | | | | | | 12.2 | 16.9 | | (17.7*) | | | |
| Resorcin | 55.1 | | 5.4 | | | | | | | | | | | | |
| Bisphenol-A | 114.2 | | | 11.6 | | | | 7.7 | | | | | | | |
| Bisphenol-S | 125.1 | | | | 12.9 | | | | | | | | | | |
| Phenolphthalein | 159.2 | | | | | 15.9 | | | | | | | | | |
| Bis-OK5 | ca. 200 | | | | | | 20.2 | | | | | | | | |
| Diole | OH-Zahl [mg/g KOH] | | | | | | | | | | | | | | |
| Priplast 2033 | 207.0 | | | | | | | | | | | | 24.2 | | |
| TCD-DM | 572.0 | | | | | | | | | | | | | 9.9 | |
| Bisphenol-A/H | 467.2 | | | | | | | | | | | | | | 12.2 |
| Epoxide | OH-Zahl [mg/g KOH] | | | | | | | | | | | | | | |
| Trimethylol-propan-di/triglycidylether | 101.0 | 78.1 | 65.7 | 79.5 | 74.9 | 74.9 | 74.4 | 50.3 | 53.0 | 76.9 | 117.5 | 117.5 | 75.7 | 69.6 | 70.2 |
| DGEBA | 13.5 | 82.9 | 82.9 | 82.9 | 82.9 | 82.0 | 82.9 | 78.0 | 78.0 | 82.5 | 82.9 | 82.9 | 82.9 | 82.0 | 82.9 |
| Summe | [g] | 426.2 | 401.5 | 421.5 | 418.2 | 418.7 | 425 | 369.9 | 377.1 | 423.1 | 447.9 | 447.9 | 430.3 | 407.4 | 412.8 |
| End-EP-Gehatt | Epoxidgehalt [eq/kg] | 2.51 | 2.43 | 2.55 | 2.53 | 2.57 | 2.51 | 2.25 | 2.31 | 2.52 | 3.15 | 3.06 | 2.42 | 2.48 | 2.51 |

| | | | | | | | | | | | | | | | |
|---|---|---|---|---|---|---|---|---|---|---|---|---|---|---|---|
| *) Bisphenol-M wurde erst nach der Epoxyterminierung des Prepolymers eingelöst | | | | | | | | | | | | | | | |

### Thixotropiermittel C

Als Beispiel für ein Thixotropiermittel **C** auf Basis eines Harnstoffderivates in einem nicht-diffundierenden Trägermaterial wurde ein Thixotropiermittel **C** gemäss Patentanmeldung EP 1 152 019 A1 in einem blockierten Polyurethan-Prepolymer mit oben erwähnten Rohstoffen hergestellt:

### Trägermaterial: Blockiertes PolyurethanPrepolymer C1:

600.0 g eines Polyetherpolyols (3000 Dalton; OH-Zahl 57 mg/g KOH) wurden unter Vakuum und Rühren bei 90°C mit 140.0 g IPDI zum Isocyanat-terminierten Prepolymer umgesetzt, bis der Isocyanatgehalt konstant blieb. Anschliessend wurden die freien Isocyanatgruppen mit Caprolactam (2% Überschuss) blockiert.

### Harnstoffderivat (HSD1) in blockiertem Polyurethan-Prepolymer:

Unter Stickstoff und leichtem Wärmen wurden 68.7 g MDI-Flocken in 181.3 g des oben beschriebenen blockierten Prepolymeres eingeschmolzen. Danach wurden während zwei Stunden unter Stickstoff und schnellem Rühren 40.1 g N-Butylamin gelöst in 219.9 g des oben beschriebenen blockierten Prepolymers zugetropft. Nach Beendigung der Zugabe der Aminlösung wurde die weisse Paste für weitere 30 Minuten weitergerührt. So wurde nach dem Abkühlen eine weisse, weiche Paste erhalten, welche einen freien Isocyanatgehalt von < 0.1 % aufwies (Anteil Harnstoffderivat ca. 20%).

### Beispiel-Zusammensetzungen

Als Beispiele wurden diverse Klebstoffzusammensetzungen gemäss Tabelle 3 und 4 hergestellt.
Als Vergleich zu den erfindungsgemässen Beispielzusammensetzungen ***Z-01*** bis ***Z-09*** wurden als nicht erfindungsgemässe Beispiele ***Ref-01*** der hochstrukturelle Epoxidklebstoff Betamate^{®}-1493 (kommerziell erhältlich von Dow-Automotive, Freienbach, Schweiz), ***Ref-02*** und ***Ref-03,*** sowie ***X-01*** bis ***X-04*** herangezogen.

Die Klebstoffe wurden nach Applikation auf elektrolytisch verzinktem Stahl (eloZn) bei 50°C während 30 Minuten im Ofen bei 180°C ausgehärtet. Alle Prüfungen erfolgten einen Tag nach Abkühlung der Verklebung auf Raumtemperatur.

**Tabelle 3. Erfindungsgemässe Zusammensetzungen.**

| | ***Z-01*** | ***Z-02*** | ***Z-03*** | ***Z-04*** | ***Z-05*** | ***Z-06*** | ***Z-07*** | ***Z-08*** | ***Z-09*** |
|---|---|---|---|---|---|---|---|---|---|
| **A-VM1** [g] | 55.6 | 55.6 | 55.6 | 55.6 | 55.6 | 55.6 | 55.6 | 55.6 | 55.6 |
| ***B-01*** [g] | 36.0 | | | | | | | | |
| ***B-02*** [g] | | 36.0 | | | | | | | |
| ***B-03*** [g] | | | 36.0 | | | | | | |
| ***B-04*** [g] | | | | 36.0 | | | | | |
| ***B-05*** [g] | | | | | 36.0 | | | | |
| ***B-06*** [g] | | | | | | 36.0 | | | |
| ***B-07*** [g] | | | | | | | 36.0 | | |
| ***B-08*** [g] | | | | | | | | 36.0 | |
| ***B-09*** [g] | | | | | | | | | 36.0 |
| ***C*** [g] | 10.0 | 10.0 | 10.0 | 10.0 | 10.0 | 10.0 | 10.0 | 10.0 | 10.0 |
| Dicyanamid **(*D*)** [g] | 4.5 | 4.5 | 4.5 | 4.5 | 4.5 | 4.5 | 4.4 | 4.4 | 4.6 |
| Füllstoffgemisch(***E***) [g] | 22.0 | 22.0 | 22.0 | 22.0 | 22.0 | 22.0 | 22.0 | 22.0 | 22.0 |
| Hexandioldiglycidylether **(*F*)** [g] | 1.5 | 1.5 | 1.5 | 1.5 | 1.5 | 1.5 | 1.5 | 1.5 | 1.5 |
| ED-506**(*F*)** [g] | 2.4 | 2.4 | 2.4 | 2.4 | 2.4 | 2.4 | 2.4 | 2.4 | 2.4 |
| ZSF [MPa] | 19.0 | 19.6 | 20.6 | 21.5 | 18.5 | 20.7 | 19.0 | 19.7 | 22.5 |
| BE¹ bei 50°C [J] | 13.7 | 14.7 | 14.2 | 15.6 | 12.9 | 15.2 | 14.2 | 12.1 | 15.3 |
| BE¹ bei 23°C [J] | 14.5 | 15.0 | 14.2 | 15.6 | 13.3 | 14.9 | 14.4 | 14.5 | 15.8 |
| BE¹ bei 0°C [J] | 13.7 | 14.1 | 12.9 | 15.1 | 14.5 | 14.4 | 15.2 | 13.2 | 13.2 |
| BE¹bei-20°C[J] | 11.9 | 11.6 | 11.1 | 12.9 | 11.5 | 11.7 | 13.3 | 12.7 | 13.2 |
| BE¹ bei -40°C [J] | 10.7 | 9.9 | 10.2 | 10.0 | 9.1 | 9.6 | 10.4 | 10.9 | 11.9 |

| | | | | | | | | | |
|---|---|---|---|---|---|---|---|---|---|
| ¹BE= Bruchenergie | | | | | | | | | |

**Tabelle 4. Referenz- und Gegenbeispiele.**

| | ***Ref-*01** | ***Ref-*02** | ***Ref-*03** | ***X-01*** | ***X-02*** | ***X-03*** | ***X-04*** |
|---|---|---|---|---|---|---|---|
| **A-VM1** [g] | | 55.6 | 55.6 | 55.6 | 55.6 | 55.6 | 55.6 |
| ***P-01*** [g] | | 18.0 | 36.0 | | | | |
| ***P-02*** [g] | | | | 36.0 | | | |
| ***P-03*** [g] | | | | | 36.0 | | |
| ***P-04*** [g] | | | | | | 36.0 | |
| ***P-05*** [g] | | | | | | | 36.0 |
| ***C*** [g] | | 21.0 | 10.0 | 10.0 | 10.0 | 10.0 | 10.0 |
| Dicyanamid *(**D**)* [g] | | 4.0 | 4.6 | 4.6 | 4.6 | 4.5 | 4.5 |
| Füllstoffgemisch(***E***) [g] | | 22.0 | 22.0 | 22.0 | 22.0 | 22.0 | 22.0 |
| Hexandioldiglycidylether (***F***) [g] | | 1.5 | 1.5 | 1.5 | 1.5 | 1.5 | 1.5 |
| ED-506**(*F*)** [g] | | 2.4 | 2.4 | 2.4 | 2.4 | 2.4 | 2.4 |
| ZSF [MPa] | 19.9 | 19.8 | 20.3 | 13.2 | 21.0 | 19.6 | 19.9 |
| BE¹ bei 50°C [J] | 18.0 | 14.3 | 13.8 | 12.9 | 9.0 | 9.7 | 15.0 |
| BE¹ bei 23°C [J] | 17.8 | 14.4 | 13.6 | 13.4 | 12.5 | 10.1 | 13.8 |
| BE¹ bei 0°C [J] | 16.2 | 14.0 | 12.5 | 11.6 | 7.2 | 10.4 | 12.0 |
| BE¹ bei -20°C [J] | 4.2 | 11.9 | 10.3 | 10.1 | 5.3 | 8.7 | 11.6 |
| BE¹ bei -40°C [J] | 0.5 | 6.0 | 5.5 | 2.9 | 0.6 | 2.3 | 0.4 |

| | | | | | | | |
|---|---|---|---|---|---|---|---|
| ¹BE= Bruchenergie | | | | | | | |

### Prüfmethoden:

### Zugscherfestigkeit (ZSF) (DIN EN 1465)

Die Probekörper wurden mit elektrolytisch verzinktem Stahl (eloZn) mit dem Mass 100 x 25 x 0.8mm hergestellt, dabei betrug die Klebfläche 25 x 10mm bei einer Schichtdicke von 0.3mm. Gehärtet wurde 30 Min. bei 180°C. Die Zuggeschwindigkeit betrug 10mm/Min.

### Schlagschälarbeit (lS0 11343)

Die Probekörper wurden mit elektrolytisch verzinktem Stahl (eloZn) mit dem Mass 90 x 25 x 0.8mm hergestellt, dabei betrug die Klebfläche 25 x 30mm bei einer Schichtdicke von 0.3mm. Gehärtet wurde 30 Min. bei 180°C. Die Zuggeschwindigkeit betrug 2 m/s. Als Bruchenergie in Joule wird die Fläche unter der Messkurve (von 25% bis 90%, gemäss DIN 11343) angegeben.

### Resultate:

Die Resultate der Klebstoffformulierungen in Tabellen 3 und 4 zeigen, dass mit den erfindungsgemässen Zusammensetzungen **(*Z-01*** bis ***Z-09)*** die Kombination von hoher Festigkeit und hoher Schlagzähigkeit sowohl bei Raumtemperatur wie auch bei tiefen Temperaturen bis -40°C erreicht werden kann.

Das Referenzbeispiel ***Ref-01*** (Betamate^{®}-1493, Dow Automotive) zeigt zwar gute Schlagzähigkeitsfestigkeiten bei Temperaturen über 0°C, weist aber bei tiefen Temperaturen, d.h. unter 0°C im Vergleich zu den erfindungsgemässen Klebstoffen bedeutend tiefere Werte auf.
Das Referenzbeispiel ***Ref-02*** enthält mit Polymer ***P-01*** ein Epoxidgruppen-terminiertes Polymer ohne phenolische Strukturelemente. Dieses Beispiel zeigt zwar im Vergleich zu ***Ref-01*** erheblich verbesserte Schlagzähigkeitswerte bei Temperaturen bis -20°C, diese fallen jedoch bei tieferen Temperaturen im Vergleich zu den erfindungsgemässen Zusammensetzungen markant ab.
Das Referenzbeispiel ***Ref-03*** ist mit ***Ref-02*** vergleichbar, enthält jedoch einerseits einen höheren Anteil des Epoxidgruppen-terminierten Polymers ***P-01*** und andererseits einen tieferen Gehalt an Thixotropiermittel C. Die erhaltenen Werte sind vergleichbar mit denjenigen, welche mit der Klebstoff-Formulierung ***Ref-02*** erhalten wurden.
Die nicht erfindungsgemässen Zusammensetzungen ***X-01*** bis ***X-04*** enthalten jeweils die Polymere ***P02*** bis ***P05. X-01*** zeigt eine speziell bei tiefen Temperaturen deutliche Reduktion der Schlagzähigkeit. ***X-02*** bis ***X-04*** weisen anstelle der Phenol-Strukturelemente solche von aliphatische Diolen herrührende Strukturelemente auf. ***X-02*** bis ***X-04*** weisen ebenfalls alle eine starke Verminderung der Schlagzähigkeit auf, insbesondere bei tiefen Temperaturen.

Die in Tabelle 3 zusammengefassten erfindungsgemässen Zusammensetzungen ***Z-01*** bis ***Z-09*** zeigen alle gute Bruchenergien. Während die übrigen mechanischen Werte wie Zugscherfestigkeit erhalten bleiben, sind im Vergleich zu den Referenzbeispielen aus Tabelle 4 besonders die Werte bei Temperaturen zwischen 0°C und -40°C stark verbessert. Dabei ist der positive Effekt im wesentlich unabhängig von den eingesetzten Diisocyanaten und Bisphenolen.

## Patentansprüche

1. Zusammensetzung umfassend mindestens ein Epoxid-Addukt **A** mit durchschnittlich mehr als einer Epoxidgruppe pro Molekül;
mindestens ein Polymer **B** der Formel (I) 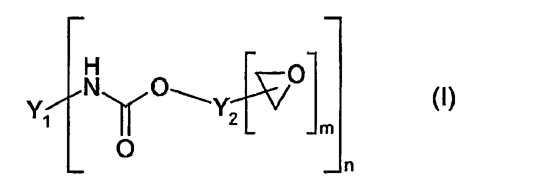 wobei
Y₁ für einen n-wertigen Rest eines mit Isocyanatgruppen terminierten linearen oder verzweigten Polyurethanprepolymeren nach dem Entfemen der endständigen Isocyanatgruppen steht;
Y₂ für einen Rest eines eine primäre oder sekundäre Hydroxylgruppe enthaltenden aliphatischen, cycloaliphatischen, aromatischen oder araliphatischen Epoxids nach dem Entfemen der Hydroxid- und Epoxidgruppen, steht;
n = 2, 3 oder 4 ist;
m = 1, 2 oder 3 ist;
und mindestens ein aromatisches Strukturelement aufweist, welches über Urethangruppen in der Polymerkette eingebunden ist;
mindestens ein Thixotropiermittel **C,** auf Basis eines Harnstoffderivates in einem nicht-diffundierenden Trägermaterial; sowie
mindestens einen Härter **D** für Epoxidharze, welcher durch erhöhte Temperatur aktiviert wird.

2. Zusammensetzung gemäss Anspruch 1, **dadurch gekennzeichnet, dass** das Epoxid-Addukt **A,** erhältlich ist aus der Reaktion
von mindestens einer Dicarbonsäure und mindestens einem Diglycidylether;
oder
von mindestens einem Bis(aminophenyl)sulfon-Isomeren oder von mindestens einem aromatischen Alkohol und mindestens einem Diglycidylether.

3. Zusammensetzung gemäss Anspruch 2, **dadurch gekennzeichnet, dass** die Dicarbonsäure eine dimere Fettsäure, insbesondere mindestens eine dimere C₄- C₂₀ Fettsäure, ist und der Diglycidylether Bisphenol-A-diglycidylether, Bisphenol-F-diglycidylether oder Bisphenol-A/F-diglycidylether ist.

4. Zusammensetzung gemäss Anspruch 2 oder 3, **dadurch gekennzeichnet, dass** der aromatische Alkohol ausgewählt ist aus der Gruppe 2,2-Bis(4-hydroxyphenyl)propan, Bis(4-hydroxyphenyl)methan, Bis(4-hydroxyphenyl)sulfon (=Bisphenol-S), Hydrochinon, Resorcin, Brenzkatechin, Naphthohydrochinon, Naphtoresorcin, Dihydroxynaphthalin, Dihydroxyanthrachinon, Dihydroxy-biphenyl, 3,3-bis(p-hydroxyphenyl)phthalide, 5,5-Bis(4-hydroxyphenyl)hexahydro-4,7-methanoindan, 4,4'-[bis-(hydroxyphenyl)-1,3-Phenylenebis-(1-Methyl-ethyliden)] (=Bisphenol-M), 4,4'-[bis-(hydroxyphenyl)-1,4-Phenylenebis-(1 -Methyl-ethyliden)] (=Bispheno)-P) sowie alle Isomeren der vorgenannten Verbindungen und der Diglycidylether Bisphenol-A-diglycidylether, Bisphenol-F-diglycidylether oder Bisphenol-A/F-diglycidylether ist.

5. Zusammensetzung gemäss einem der vorhergehenden Ansprüche, **dadurch gekennzeichnet, dass** das Polymer **B** in Epoxidharzen löslich oder dispergierbar ist.

6. Zusammensetzung gemäss einem der vorhergehenden Ansprüche, **dadurch gekennzeichnet, dass** das Polymer **B** aus der Reaktion eines Monohydroxyepoxids der Formel (II) und eines Isocyanatgruppen terminierten linearen oder verzweigten Polyurethanprepolymeren der Formel (III) 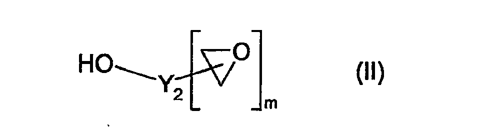 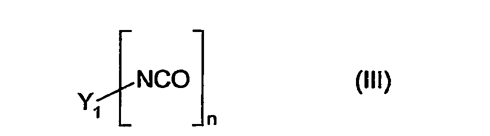 erhältlich ist.

7. Zusammensetzung gemäss Anspruch 6, **dadurch gekennzeichnet, dass** im Herstellprozess des Polyurethanprepolymers der Formel (III) mindestens ein Polyisocyanat, mindestens ein, gegebenenfalls substituiertes, Polyphenol und mindestens ein Isocyanat-reaktives Polymer verwendet wird.

8. Zusammensetzung gemäss Anspruch 7, **dadurch gekennzeichnet, dass** das der Formel (III) zugrunde liegende Isocyanat-reaktive Polymer ein Polyol ausgewählt aus der Gruppe der Polyoxyalkylenpolyole, Polyhydroxyterminerte Polybutadienpolyole, Styrol-Acrylnitril gepfropfte Polyetherpolyole, Polyhydroxyterminerte Acyrlonitril/Butadien-Copolymere, Polyesterpolyole und Polycarbonatpolyole ist.

9. Zusammensetzung gemäss Anspruch 7 oder 8, **dadurch gekennzeichnet, dass** das der Formel (III) zugrunde liegende lsocyanat-reaktive Polymer ein α,ω- Polyalkylenglykol mit C₂-C₆-Alkylengruppen oder mit gemischten C₂-C₆- Alkylengruppen, insbesondere ein Polypropylenglykol oder ein Polybutylenglykol ist.

10. Zusammensetzung gemäss einem der Ansprüche 7-9, **dadurch gekennzeichnet, dass** das der Formel (III) zugrunde liegenden Isocyanat-reaktive Polymer ein Equivalenzgewicht von 600- 6'000 g/Equivalent NCO-reaktiver Gruppe , insbesondere von 700- 2200 g/Equivalent NCO-reaktiver Gruppe besitzt.

11. Zusammensetzung gemäss einem der Ansprüche 7-9, **dadurch gekennzeichnet, dass** das Formel (III) zugrunde liegende Polyisocyanat ein Diisocyanat, bevorzugt HDI, IPDI, TMDI, MDI oder TDI, ist.

12. Zusammensetzung gemäss einem der vorhergehenden Ansprüche, **dadurch gekennzeichnet, dass** die Polymerkette des Polymers **B** gleichzeitig die Strukturelemente der Formel (IV) und (V) 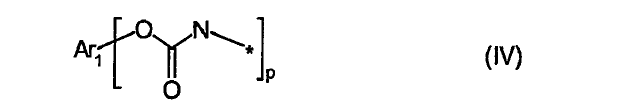 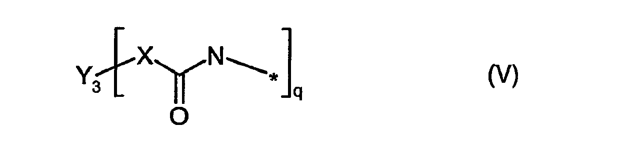 aufweist, wobei
p = 2, 3 oder 4, insbesondere p = 2 oder 3, ist;
q = 2, 3 oder 4, insbesondere q = 2 oder 3, ist;
X = S, O oder NH; insbesondere X = O ist;
Ar₁ einen p-valenten, gegebenenfalls substituierten, Arylrest, darstellt;
Y₃ einen q-wertigen Rest eines Isocyanat-reaktiven Polymers nach dem Entfemen der endständigen Amino-, Thiol- oder Hydroxylgruppen darstellt und
* die Anbindungsstelle zum Rest der Polymerkette darstellt.

13. Zusammensetzung gemäss einem der vorhergehenden Ansprüche, **dadurch gekennzeichnet, dass** der Gewichtsanteil aller Polymere **B** der Formel (I) zwischen 5 und 40 Gewichts-%, vorzugsweise zwischen 7 und 30 Gewichts-% bezogen auf das Gewicht der gesamten Zusammensetzung beträgt.

14. Zusammensetzung gemäss einem der vorhergehenden Ansprüche, **dadurch gekennzeichnet, dass** das Trägermaterial des Thixotropiermittels **C** ein blockiertes Polyurethanprepolymer ist.

15. Zusammensetzung gemäss einem der vorhergehenden Ansprüche, **dadurch gekennzeichnet, dass** das Harnstoffderivat im Thixotropiermittel **C** das Produkt der Umsetzung eines aromatischen monomeren Diisocyanaten, insbesondere 4,4'-Diphenyl-methylen-diisocyanat, mit einer aliphatischen Aminverbindung, insbesondere Butylamin ist.

16. Zusammensetzung gemäss einem der vorhergehenden Ansprüche, **dadurch gekennzeichnet, dass** der Gesamtanteil des Thixotropiermittels **C** 5-40 Gewichts-%, vorzugsweise 10 - 25 % Gewichts-%, bezogen auf das Gewicht der gesamten Zusammensetzung, beträgt.

17. Zusammensetzung gemäss einem der vorhergehenden Ansprüche, **dadurch gekennzeichnet, dass** der Anteil des Harnstoffderivats 5- 50 Gewichts-%, vorzugsweise 15-30 Gewichts-%, bezogen auf das Gewicht des Thixotropiermittels C, beträgt.

18. Zusammensetzung gemäss einem der vorhergehenden Ansprüche, **dadurch gekennzeichnet, dass** der Härter **D** ein latenter Härter ist ausgewählt aus der Gruppe umfassend Dicyandiamid, Guanamine, Guanidine und Aminoguanidine.

19. Zusammensetzung gemäss einem der vorhergehenden Ansprüche, **dadurch gekennzeichnet, dass** der Gesamtanteil des Härters **D** 1 - 10 Gewichts-%, vorzugsweise 2-8 Gewichts%, bezogen auf das Gewicht der gesamten Zusammensetzung, beträgt.

20. Zusammensetzung gemäss einem der vorhergehenden Ansprüche, **dadurch gekennzeichnet, dass** zusätzlich mindestens ein Füllstoff **E** vorhanden ist.

21. Zusammensetzung gemäss Anspruch 20, **dadurch gekennzeichnet, dass** der Gesamtanteil des Füllstoffs **E** 5- 30 Gewichts-%, vorzugsweise 10-25 Gewichts-%, bezogen auf das Gewicht der gesamten Zusammensetzung, beträgt.

22. Zusammensetzung gemäss einem der vorhergehenden Ansprüche, **dadurch gekennzeichnet, dass** zusätzlich mindestens ein epoxidgruppentragender Reaktiwerdünner **F** vorhanden ist.

23. Zusammensetzung gemäss einem der vorhergehenden Ansprüche, **dadurch gekennzeichnet, dass** die Zusammensetzung nach Aushärtung eine Tieftemperatur-Bruchenergie gemessen nach DIN 11343 von mehr als 10 J bei -20°C und von mehr als 7 J bei -40°C und bevorzugt von mehr als 11 J bei -20°C und von mehr als 9 J bei -40°C, aufweist.

24. Verwendung einer Zusammensetzung gemäss einem der Ansprüche 1 - 23 als einkomponentiger Klebstoff.

25. Verwendung eines Epoxidgruppen-terminierten Schlagzähigkeitsmodifikators der Formel (I) in einem zweikomponentigen Klebstoff, **dadurch gekennzeichnet, dass** dieser Schlagzähigkeitsmodifikator Bestandteil der ersten Komponente ist und mindestens ein Polyamin oder mindestens ein Polymerkaptan Bestandteil der zweiten Komponente ist; 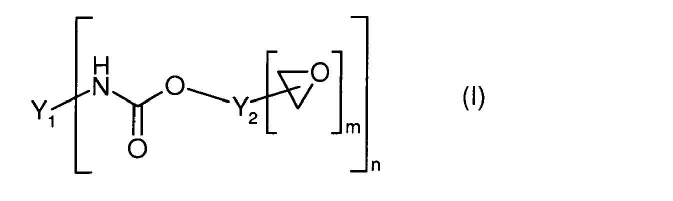 wobei
Y₁ für einen n-wertigen Rest eines mit Isocyanatgruppen terminierten linearen oder verzweigten Polyurethanprepolymeren nach dem Entfernen der endständigen Isocyanatgruppen steht;
Y₂ für einen Rest eines eine primäre oder sekundäre Hydroxylgruppe enthaltenden aliphatischen, cycloaliphatischen, aromatischen oder araliphatischen Epoxids nach dem Entfernen der Hydroxid- und Epoxidgruppen, steht;
n= 2, 3 oder 4 ist;
m = 1, 2 oder 3 ist;
und mindestens ein aromatisches Strukturelement aufweist, welches über Urethangruppen in der Polymerkette eingebunden ist.

26. Verwendung gemäss Anspruch 25, **dadurch gekennzeichnet, dass** die Polymerkette des Schlagzähigkeitsmodifikator gleichzeitig die Strukturelemente der Formel (lV) und (V) 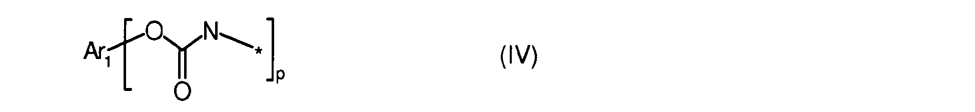 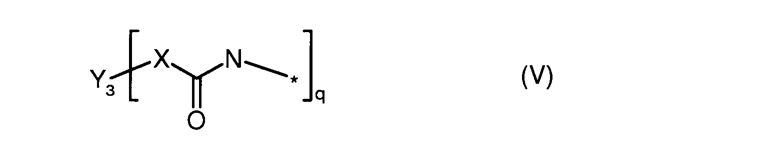 aufweist, wobei
p = 2, 3 oder 4, insbesondere p = 2 oder 3, ist;
q = 2, 3 oder 4, insbesondere q = 2 oder 3, ist;
X = S, O oder NH; insbesondere X = O, ist;
Ar₁ einen p-valenten, gegebenenfalls substituierten, Arylrest, darstellt;
Y₃ einen q-wertigen, gegebenenfalls kettenverlängerten, Rest eines Isocyanat-reaktiven Polymers nach dem Entfernen der endständigen Amino-, Thiol- oder Hydroxylgruppen darstellt und
* die Anbindungsstelle zum Rest der Polymerkette darstellt.

27. Verwendung gemäss Anspruch 25 oder 26, **dadurch gekennzeichnet, dass** der Schlagzähigkeitsmodifikator aus der Reaktion eines Monohydroxyepoxids der Formel (II) mit einem Isocyanatgruppen terminierten linearen oder verzweigten Polyurethanprepolymeren der Formel (III) erhältlich ist, und dass in der Herstellung dieses Polyurethanprepolmers mindestens ein Polyisocyanat und mindestens ein Polyphenol und mindestens ein Isocyanat-reaktives Polymer eingesetzt werden.

28. Verwendung gemäss einem der Ansprüche 24 bis 27, **dadurch gekennzeichnet, dass** der Klebstoff für das Verkleben von hitzestabilen Materialien, insbesondere von Metallen, verwendet wird.

29. Verwendung gemäss einem der Ansprüche 24 bis 28, **dadurch gekennzeichnet, dass** der Klebstoff als Rohbauklebstoff im Automobilbau verwendet wird.

30. Verfahren zum Verkleben von hitzestabilen Materialien, insbesondere von Metallen, **dadurch gekennzeichnet, dass** diese Materialien mit einer Zusammensetzung gemäss einem der Ansprüche 1 - 23 kontaktiert werden und einen späteren Schritt des Aushärtens bei einer Temperatur von 100 - 220 °C, vorzugsweise 120 - 200°C umfasst.

## Claims

1. A composition comprising
at least one epoxide adduct **A** having on average more than one epoxide group per molecule;
at least one polymer **B** of the formula (I) 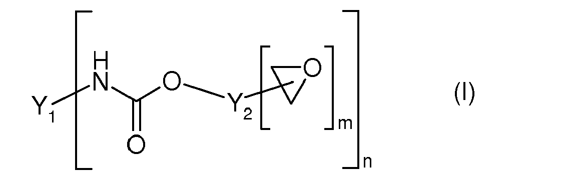 in which
Y₁ is an n-valent radical of a linear or branched polyurethane prepolymer terminated with isocyanate groups after removal of the terminal isocyanate groups;
Y₂ is a radical of an aliphatic, cycloaliphatic, aromatic or araliphatic epoxide containing a primary or secondary hydroxyl group after removal of the hydroxide and epoxide groups;
n is 2, 3 or 4 ;
m is 1, 2 or 3 ;
and has at least one aromatic structural element which is bound in the polymer chain via urethane groups;
at least one thixotropic agent **C** based on a urea derivative in a nondiffusing carrier material; and
at least one curing agent **D** for epoxy resins which is activated by elevated temperature.

2. The composition as claimed in claim 1, **characterized in that** the epoxide adduct **A** is obtainable from the reaction
of at least one dicarboxylic acid and at least one diglycidyl ether;
or
of at least one bis(aminophenyl) sulfone isomer or of at least one aromatic alcohol and at least one diglycidyl ether.

3. The composition as claimed in claim 2, **characterized in that** the dicarboxylic acid is a dimeric fatty acid, in particular at least one dimeric C₄-C₂₀ fatty acid, and the diglycidyl ether is bisphenol A diglycidyl ether, bisphenol F diglycidyl ether or bisphenol A/F diglycidyl ether.

4. The composition as claimed in claim 2 or 3, **characterized in that** the aromatic alcohol is selected from the group consisting of 2,2-bis(4-hydroxyphenyl)propane, bis(4-hydroxyphenyl)methane, bis(4-hydroxyphenyl) sulfone (= bisphenol S), hydroquinone, resorcinol, pyrocatechol, naphthohydroquinone, naphthoresorcinol, dihydroxynaphthalene, dihydroxyanthraquinone, dihydroxybiphenyl, 3,3-bis(p-hydroxyphenyl)phthalides, 5,5-bis(4-hydroxyphenyl)hexahydro-4,7-methanoindane, 4,4'-[bis(hydroxyphenyl)-1,3-phenylenebis(1-methylethylidene)] (= bisphenol M), 4,4'-[bis(hydroxyphenyl)-1,4-phenylenebis(1-methylethylidene)] (= bisphenol P) and all isomers of the abovementioned compounds, and the diglycidyl ether is bisphenol A diglycidyl ether, bisphenol F diglycidyl ether or bisphenol A/F diglycidyl ether.

5. The composition as claimed in any of the preceding claims, **characterized in that** the polymer **B** is soluble or dispersible in epoxy resins.

6. The composition as claimed in any of the preceding claims, **characterized in that** the polymer **B** is obtainable from the reaction of a monohydroxyepoxide of the formula (II) and of a linear or branched polyurethane prepolymer terminated with isocyanate groups and of the formula (III)

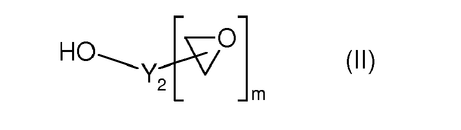

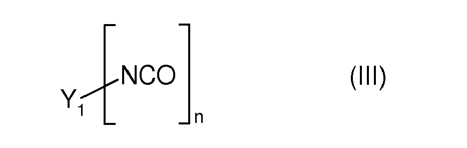

7. The composition as claimed in claim 6, **characterized in that**, in the process for the preparation of the polyurethane prepolymer of the formula (lll), at least one polyisocyanate, at least one, optionally substituted, polyphenol and at least one isocyanate-reactive polymer are used.

8. The composition as claimed in claim 7, **characterized in that** the isocyanate-reactive polymer of the formula (III) is a polyol selected from the group consisting of the polyoxyalkylenepolyols, polyhydroxy-terminated polybutadienepolyols, styrene/acrylonitrile-grafted polyetherpolyols, polyhydroxy-terminated acrylonitrile/butadiene copolymers, polyesterpolyols and polycarbonatepolyols.

9. The composition as claimed in claim 7 or 8, **characterized in that** the isocyanate-reactive polymer of the formula (III) is an α,ω-polyalkylene glycol having C₂-C₆-alkylene groups or having mixed C₂-C₆-alkylene groups, in particular a polypropylene glycol or a polybutylene glycol.

10. The composition as claimed in any of claims 7-9, **characterized in that** the isocyanate-reactive polymer of the formula (III) has an equivalent weight of 600 - 6000 g/equivalent of NCO-reactive groups, in particular of 700 - 2200 g/equivalent of NCO-reactive group.

11. The composition as claimed in any of claims 7-9, **characterized in that** the polyisocyanate of the formula (III) is a diisocyanate, preferably HDI, IPDI, TMDI, MDI or TDI.

12. The composition as claimed in any of the preceding claims, **characterized in that** the polymer chain of the polymer **B** simultaneously has the structural elements of the formulae (lV) and (V) 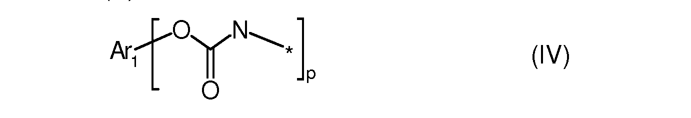 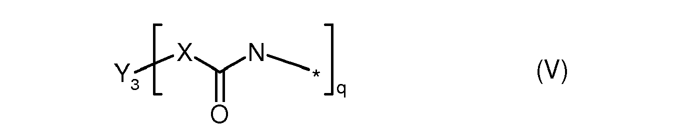 in which
p is 2, 3 or 4, in particular p is 2 or 3;
q is 2, 3 or 4, in particular q is 2 or 3;
X is S, O or NH; in particular X is O;
Ar₁ is a p-valent, optionally substituted, aryl radical;
Y₃ is a q-valent radical of an isocyanate-reactive polymer after removal of the terminal amino, thiol or hydroxyl groups and
* is the linkage point to the remainder of the polymer chain.

13. The composition as claimed in any of the preceding claims, **characterized in that** the proportion by weight of all polymers **B** of the formula (I) is from 5 to 40% by weight, preferably from 7 to 30% by weight, based on the weight of the total composition.

14. The composition as claimed in any of the preceding claims, **characterized in that** the carrier material of the thixotropic agent **C** is a blocked polyurethane prepolymer.

15. The composition as claimed in any of the preceding claims, **characterized in that** the urea derivative in the thixotropic agent **C** is a product of the reaction of an aromatic monomeric diisocyanate, in particular 4,4'-diphenylmethylene diisocyanate, with an aliphatic amine compound, in particular butylamine.

16. The composition as claimed in any of the preceding claims, **characterized in that** the total proportion of the thixotropic agent **C** is 5 - 40% by weight, preferably 10 - 25% by weight, based on the weight of the total composition.

17. The composition as claimed in any of the preceding claims, **characterized in that** the proportion of the urea derivative is 5 - 50% by weight, preferably 15 - 30% by weight, based on the weight of the thixotropic agent **C.**

18. The composition as claimed in any of the preceding claims, **characterized in that** the curing agent **D** is a latent curing agent selected from the group consisting of dicyandiamide, guanamines, guanidines and aminoguanidines.

19. The composition as claimed in any of the preceding claims, **characterized in that** the total proportion of the curing agent **D** is 1 - 10% by weight, preferably 2 - 8% by weight, based on the weight of the total composition.

20. The composition as claimed in any of the preceding claims, **characterized in that** at least one filler **E** is additionally present.

21. The composition as claimed in claim 20, **characterized in that** the total proportion of the filler **E** is 5 - 30% by weight, preferably 10 - 25% by weight, based on the weight of the total composition.

22. The composition as claimed in any of the preceding claims, **characterized in that** at least one reactive diluent **F** carrying epoxide groups is additionally present.

23. The composition as claimed in any of the preceding claims, **characterized in that** the composition, after curing, has a low-temperature fracture energy, measured according to DIN 11343, of more than 10 J at -20°C and of more than 7 J at -40°C and preferably of more than 11 J at -20°C and of more than 9 J at -40°C.

24. Use of a composition as claimed in any of the claims 1-23 as one component adhesive.

25. Use of an impact strength modifier of the formula (I) terminated by epoxide groups in a two component adhesive, **characterized in that** said impact strength modifier is part of the first component and that at least a polyamine or at least a polymercaptone is part of the second component 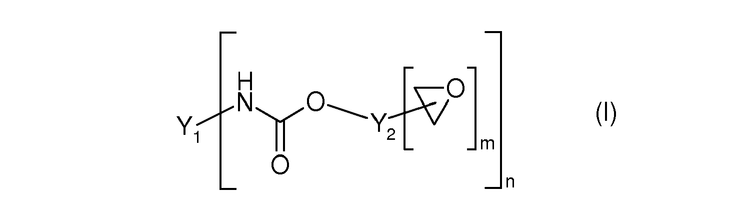 in which
Y₁ is an n-valent radical of a linear or branched polyurethane prepolymer terminated with isocyanate groups after removal of the terminal isocyanate groups;
Y₂ is a radical of an aliphatic, cycloaliphatic, aromatic or araliphatic epoxide containing a primary or secondary hydroxyl group after removal of the hydroxide and epoxide groups;
n is 2, 3 or 4;
m is 1, 2 or 3;
and has at least one structural element which is bound in the polymer chain via urethane groups.

26. Use as claimed in claim 25, **characterized in that** the polymer chain of the impact strength modifier simultaneously has structural elements of the formulae (IV) and (V) 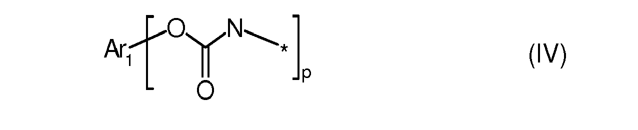 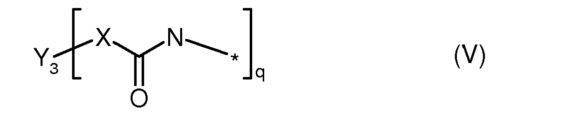 in which
p is 2, 3 or 4, in particular p is 2 or 3;
q is 2, 3 or 4, in particular q is 2 or 3;
X is S, O or NH; in particular X is O;
Ar₁ is a p-valent, optionally substituted aryl radical;
Y₃ is a q-valent, optionally chain-extended, radical of an isocyanate-reactive polymer after removal of the terminal amino, thiol or hydroxyl groups and
* is the linkage point to the remainder of the polymer chain.

27. Use as claimed in claim 25 or 26, **characterized in that** the impact strength modifier is obtainable from the reaction of a monohydroxyepoxide of the formula (II) with a linear or branched polyurethane prepolymer terminated with isocyanate groups and of the formula (III), and **in that** at least one polyisocyanate and at least one polyphenol and at least one isocyanate-reactive polymer are used in the preparation of this polyurethane prepolymer.

28. The use as claimed in claims any of 24 or 27, **characterized in that** the adhesive is used for the adhesive bonding of heat-stable materials, in particular of metals.

29. The use as claimed in any of claims 24 to 28, **characterized in that** the adhesive is used as a body-shell construction adhesive in automotive construction.

30. A method for the adhesive bonding of heat-stable materials, in particular of metals, **characterized in that** these materials are brought into contact with a composition as claimed in any of claims 1 - 23 and comprises a subsequent step of curing at a temperature of 100 - 220°C, preferably 120 - 200°C.

## Revendications

1. Composition comprenant
au moins un adduit époxyde A, comportant en moyenne plus d'un groupe époxy par molécule ;
au moins un polymère B de formule (I) 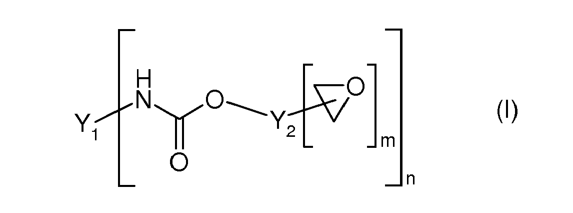
dans laquelle
Y₁ est un radical n-valent d'un prépolymère de polyuréthane à chaîne droite ou ramifiée, terminé par des groupes isocyanate, après élimination des groupes isocyanate terminaux ;
Y₂ est un radical d'un époxyde aliphatique, cycloaliphatique, aromatique ou araliphatique contenant un groupe hydroxyle primaire ou secondaire, après élimination des groupes hydroxy et époxy ;
n = 2, 3 ou 4 ;
m = 1, 2 ou 3 ;
et comprend au moins un élément structurel aromatique qui est incorporé dans la chaîne polymère par l'intermédiaire de groupes uréthane ;
au moins un agent thixotrope C, à base d'un dérivé de l'urée, dans un matériau support non diffusant ;
ainsi
qu'au moins un durcisseur D pour résines époxydes, qui est activé par une température accrue.

2. Composition selon la revendication 1, **caractérisée en ce que** l'adduit époxyde A peut être obtenu à partir de la réaction
d'au moins un acide dicarboxylique et d'au moins un éther diglycidylique ;
ou
d'au moins un isomère de bis(aminophényl)sulfone ou d'au moins un alcool aromatique et d'au moins un diglycidyléther.

3. Composition selon la revendication 2, **caractérisée en ce que** l'acide dicarboxylique est un acide gras dimère, en particulier au moins un acide gras dimère en C₄-C₂₀, et l'éther diglycidylique est l'éther diglycidylique du bisphénol A, l'éther diglycidylique du bisphénol F ou l'éther diglycidylique du bisphénol A/F.

4. Composition selon la revendication 2 ou 3, **caractérisée en ce que** l'alcool aromatique est choisi dans le groupe composé de 2,2-bis(4-hydroxyphényl)propane, bis(4-hydroxyphényl)méthane, bis(4-hydroxyphényl)sulfone (= bisphénol S), hydroquinone, résorcinol, pyrocatéchol, naphtohydroquinone, naphtorésorcinol, dihydroxy-naphtalène, dihydroxyanthraquinone, dihydroxybiphényle, 3,3-bis(p-hydroxyphényl)phtalide, 5,5-bis(4-hydroxy-phényl)hexahydro-4,7-méthanoindane, 4,4'-[bis(hydroxy-phényl)-1,3-phénylènebis(1-méthyl-éthylidène)] (= bisphénol M), 4,4'-[bis(hydroxyphényl)-1,4-phénylène-bis(1-méthyl-éthylidène)] (= bisphénol P), ainsi que tous les isomères des composés mentionnés ci-dessus, et l'éther diglycidylique est l'éther diglycidylique du bisphénol A, l'éther diglycidylique du bisphénol F ou l'éther diglycidylique du bisphénol A/F.

5. Composition selon l'une des revendications précédentes, **caractérisée en ce que** le polymère B est soluble ou dispersible dans les résines époxydes.

6. Composition selon l'une des revendications précédentes, **caractérisée en ce que** le polymère B peut être obtenu par la réaction d'un monohydroxyépoxyde de formule (II) et d'un prépolymère de polyuréthane à chaîne droite ou ramifiée, terminé par des groupes isocyanate, de formule (III)

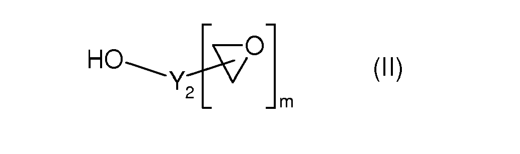

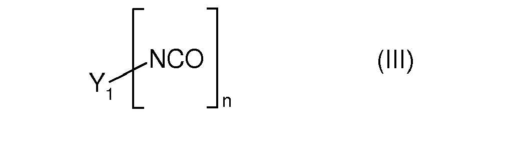

7. Composition selon la revendication 6, **caractérisée en ce que**, dans le procédé de préparation du prépolymère de polyuréthane de formule (III), on utilise au moins un polyisocyanate, au moins un polyphénol, éventuellement substitué, et au moins un polymère réactif vis-à-vis des isocyanates.

8. Composition selon la revendication 7, **caractérisée en ce que** le polymère réactif vis-à-vis des isocyanates et à base de la formule (III) est un polyol choisi dans le groupe des polyoxyalkylène-polyols, des polybutadiènepolyols à terminaison polyhydroxy, des polyétherpolyols à greffage styrène-acrylonitrile, des copolymères acrylonitrile/butadiène à terminaison polyhydroxy, des polyesters polyols et des polycarbonatepolyols.

9. Composition selon la revendication 7 ou 8, **caractérisée en ce que** le polymère réactif vis-à-vis des isocyanates et à base de la formule (III) est un α,ω-polyalkylèneglycol ayant des groupes alkylène en C₂ à C₆ ou des groupes alkylène mélangés en C₂ à C₆, en particulier un polypropylèneglycol ou un polybutylèneglycol.

10. Composition selon l'une des revendications 7 à 9, **caractérisée en ce que** le polymère réactif vis-à-vis des isocyanates et à base de la formule (III) présente une masse équivalente de 600 à 6000 g/équivalent de groupes réactifs vis-à-vis des groupes NCO, en particulier de 700 à 2200 g/équivalent de groupes réactifs vis-à-vis des groupes NCO.

11. Composition selon l'une des revendications 7 à 9, **caractérisée en ce que** le polyisocyanate à base de la formule (III) est un diisocyanate, de préférence le HDI, l'IPDI, le TMDI, le MDI ou le TDI.

12. Composition selon l'une des revendications précédentes, **caractérisée en ce que** la chaîne polymère du polymère B comprend simultanément les éléments structuraux de formule (IV) et (V) 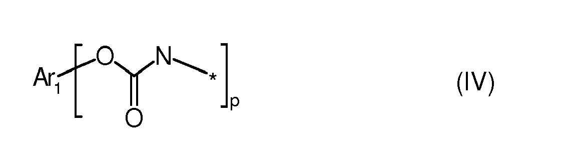 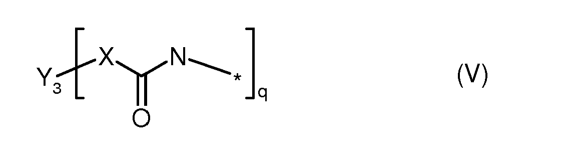 où
p = 2, 3 ou 4, en particulier p = 2 ou 3 ;
q = 2, 3 ou 4, en particulier q = 2 ou 3 ;
X = S, O ou NH ; en particulier X = O ;
Ar₁ est un radical aryle p-valent, éventuellement substitué ;
Y₃ est un radical q-valent d'un polymère réactif vis-à-vis des isocyanates, après élimination des groupes amino, thiol ou hydroxy terminaux, et
* représente le site de liaison au radical de la chaîne polymère.

13. Composition selon l'une des revendications précédentes, **caractérisée en ce que** la proportion pondérale de l'ensemble des polymères B de formule (I) est comprise entre 5 et 40 % en poids, de préférence entre 7 et 30 % en poids par rapport au poids de la totalité de la composition.

14. Composition selon l'une des revendications précédentes, **caractérisée en ce que** le matériau support de l'agent thixotrope C est un prépolymère de polyuréthane bloqué.

15. Composition selon l'une des revendications précédentes, **caractérisée en ce que** le dérivé de l'urée se trouvant dans l'agent thixotrope C est le produit de la réaction d'un diisocyanate monomère aromatique, en particulier le diisocyanato-4,4'-diphénylméthylène, avec un composé aminé aliphatique, en particulier la butylamine.

16. Composition selon l'une des revendications précédentes, **caractérisée en ce que** la proportion totale de l'agent thixotrope C est de 5 à 40 % en poids, de préférence de 10 à 25 % en poids, par rapport au poids de la totalité de la composition.

17. Composition selon l'une des revendications précédentes, **caractérisée en ce que** la proportion du dérivé de l'urée est de 5 à 50 % en poids, de préférence de 15 à 30 % en poids, par rapport au poids de l'agent thixotrope C.

18. Composition selon l'une des revendications précédentes, **caractérisée en ce que** le durcisseur D est un durcisseur latent, choisi dans le groupe comprenant le dicyanodiamide, les guanamines, les guanidines et les aminoguanidines.

19. Composition selon l'une des revendications précédentes, **caractérisée en ce que** la proportion totale du durcisseur D est de 1 à 10 % en poids, de préférence de 2 à 8 % en poids, par rapport au poids de la totalité de la composition.

20. Composition selon l'une des revendications précédentes, **caractérisée par** la présence supplémentaire d'au moins une matière de charge E.

21. Composition selon la revendication 20, **caractérisée en ce que** la proportion totale de la matière de charge E est de 5 à 30 % en poids, de préférence de 10 à 25 % en poids, par rapport au poids de la totalité de la composition.

22. Composition selon l'une des revendications précédentes, **caractérisée par** la présence supplémentaire d'au moins un diluant réactif F portant des groupes époxy.

23. Composition selon l'une des revendications précédentes, **caractérisée en ce que** la composition, après durcissement, présente une énergie de rupture à basse température, mesurée selon DIN 11343, supérieure à 10 J à -20°C, et supérieure à 7 J à -40°C, et de préférence supérieure à 11 J à -20°C et supérieure à 9 J à -40°C.

24. Utilisation d'une composition selon l'une des revendications 1 à 23 en tant qu'adhésif monocomposant.

25. Utilisation d'un agent modifiant la résistance au choc, terminé par des groupes époxy, de formule (I), dans un adhésif bicomposant, **caractérisée en ce que** cet agent modifiant la résistance au choc est un constituant du premier composant, et au moins une polyamine ou au moins un polythiol est un constituant du deuxième composant ; 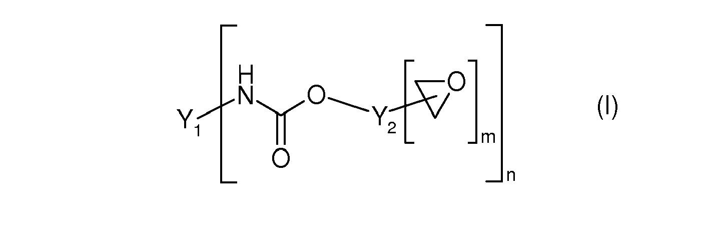 dans laquelle
Y₁ est un radical n-valent d'un prépolymère de polyuréthane à chaîne droite ou ramifiée, terminé par des groupes isocyanate, après élimination des groupes isocyanate terminaux ;
Y₂ est un radical d'un époxyde aliphatique, cycloaliphatique, aromatique ou araliphatique contenant un groupe hydroxyle primaire ou secondaire, après élimination des groupes hydroxy et époxy ;
n = 2, 3 ou 4 ;
m = 1, 2 ou 3 ;
et comprend au moins un élément structurel aromatique qui est incorporé dans la chaîne polymère par l'intermédiaire de groupes uréthane.

26. Utilisation selon la revendication 25, **caractérisée en ce que** la chaîne polymère de l'agent modifiant la résistance au choc comprend simultanément les éléments structuraux de formule (IV) et (V) 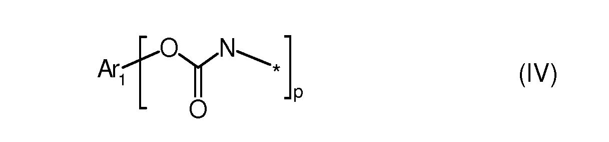 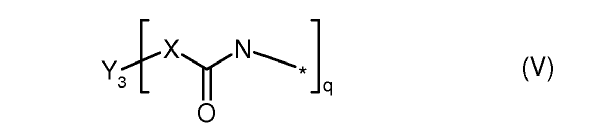 où
p = 2, 3 ou 4, en particulier p = 2 ou 3 ;
q = 2, 3 ou 4, en particulier q = 2 ou 3 ;
X = S, O ou NH ; en particulier X = O ;
Ar₁ est un radical aryle p-valent, éventuellement substitué ;
Y₃ est un radical q-valent, éventuellement à chaîne plus longue, d'un polymère réactif vis-à-vis des isocyanates, après élimination des groupes amino, thiol ou hydroxy terminaux, et
* représente le site de liaison au radical de la chaîne polymère.

27. Utilisation selon la revendication 25 ou 26, **caractérisée en ce que** l'agent modifiant la résistance au choc peut être obtenu par la réaction d'un monohydroxyépoxyde de formule (II) avec un prépolymère de polyuréthane à chaîne droite ou ramifiée, terminé par des groupes isocyanate, de formule (III), et **en ce que**, lors de la préparation de ce prépolymère de polyuréthane, on utilise au moins un polyisocyanate et au moins un polyphénol et au moins un polymère réactif vis-à-vis des isocyanates.

28. Utilisation selon l'une des revendications 24 à 27, **caractérisée en ce que** l'adhésif est utilisé pour coller des matériaux stables à la chaleur, en particulier des métaux.

29. Utilisation selon l'une des revendications 24 à 28, **caractérisée en ce que** l'adhésif est utilisé en tant qu'adhésif pour la structure de carrosserie dans la construction automobile.

30. Procédé pour coller des matériaux stables à la chaleur, en particulier des métaux, **caractérisé en ce que** ces matériaux sont mis en contact avec une composition selon l'une des revendications 1 à 23, et le procédé comprend une étape ultérieure de durcissement à une température de 100 à 220°C, de préférence de 120 à 200°C.
